# EUROPEAN PATENT APPLICATION

(11) **EP 2 239 576 A1**
(43) Date of publication of application: **13.10.2010**
(21) Application number: 09702423.6
(22) Date of filing: 16.01.2009
(51) Int. Cl.: G01N 33/574, G01N 33/577, C07K 14/47, C07K 16/18

(54) **COMPOSITION AND METHOD FOR DIAGNOSIS OR DETECTION OF GASTRIC CANCER**

(30) Priority: 17.01.2008 JP 2008008411
(71) Applicant: Toray Industries, Inc., Tokyo 103-8666 (JP)
(72) Inventor: TANAKA, Yoshinori, Kamakura-shi Kanagawa 248-8555 (JP); KOBAYASHI, Michimoto, Kamakura-shi Kanagawa 248-8555 (JP); TOMODA, Shiori, Kamakura-shi Kanagawa 248-8555 (JP); JUNG, Giman, Kamakura-shi Kanagawa 248-8555 (JP); OGAWA, Osamu, Kyoto-shi Kyoto 606-8501 (JP); NAKAMURA, Eijiro, Kyoto-shi Kyoto 606-8501 (JP)
(74) Representative: Prüfer & Partner GbR European Patent Attorneys
(86) International application number: PCT/JP2009/050524
(87) International publication number: WO 2009/091023

(57) **Abstract**

The present invention relates to a method for detection of a renal cancer, comprising measuring any one of or a plurality of polypeptides shown in SEQ ID NOS: 1-7, mutants thereof, or fragments thereof in a biological sample from a subject, and to a composition for diagnosis or detection of the renal cancer.

## Description

### TECHNICAL FIELD

The present invention relates to a composition useful for diagnosis or detection of renal cancer.

The present invention further relates to a method for detecting renal cancer using the composition.

### BACKGROUND ART

The kidney is an important organ of the urinary system, and it plays a role in filtrating blood to generate urine, thereby excreting waste products from the body. The kidney is also an important endocrine organ producing hormones such as angiotensin which controls blood pressure and erythropoietin which is an erythrocyte hematopoietic factor.

Tumors in kidneys include renal cell cancer, which occurs in adults, Wilms tumor, which occurs in children, and sarcoma as a rare tumor. Hereafter, the renal cell cancer, which is the most common type of malignant tumor, is referred to as renal cancer. The incidence of renal cancer is about 2.5 per population of 100,000. The male to female ratio for renal cancer ranges from 2 : 1 to 3 : 1, suggesting that it tends to occur more frequently in males. Among malignant urological tumors, the renal cancer is the third most common tumor, following prostate cancer and bladder cancer.

Genetic factors are known as renal cancer risk factors. In general, tobacco, fat intake, and the like are examples of the risk factors. It is also known that there is a high tumor incidence rate in patients who have received dialysis for a long time period.

In the case of renal cancer, the presence of some subjective symptoms is rare when the maximum tumor size is 5 cm or less. Renal cancer is often found by CT scan or the like upon physical examination. Hematuria, abdominal tumor, pain, and the like are observed in the case of large-sized tumors. Fever, body weight loss, anemia, or the like may occur as systemic symptoms. Polycythemia, hypertension, hypercalcemia, or the like may be rarely induced by endocrine factors. Meanwhile, spread of renal cancer to the inferior vena cava may result in venous dilatation of abdominal body surface or testicular varicocele. About 20% of renal cancer cases are found to have resulted from lung metastasis or bone metastasis. Renal cancer shows a strong tendency for the tumor to spread intravenously. Renal cancer easily metastasizes to other organs.

Examples of methods for examining renal cancer include ultrasonography, CT scan, and angiographic examination, but no specific biochemical marker therefor is known. Hence, physical examination using instruments is required.

Also, the renal cancer can be further classified pathologically. The cause of clear cell renal cancer, which accounts for about 90% of renal cancer cases, is known to be deficiency of a VHL (von-Hippel-Lindau) gene that is a tumor suppressor gene (Latif, F. et al., Science, 1993, Vol. 260, p. 1317-1320). It is known that the VHL gene deficiency results in the transcriptional activation of a hypoxia-inducible gene group via interruption of HIF-α/VHF association (Maxwell, P. et al., Nature, 1999, Vol. 399, p. 271-275). It is also known that VHL gene deficiency causes enhanced expression of genes such as VEGF and TGFβ.

Renal cancer is mainly treated by surgery. Regardless of stage, removal of the whole or part of kidney is most commonly performed when the removal is possible. If metastasis occurs, surgical removal of the kidney may be considered. A method other than surgery is the arterial embolization of the renal artery, which may be performed prior to surgery when removal is impossible or when a large tumor is removed.

When renal cancer is found relatively early, the prognosis is relatively good, such that 90% or more of early cancer cases are cured. However, treatment results for large tumors with sizes of 5 cm or larger or metastatic tumors are poor (the 5-year survival rate of all renal cell cancer cases ranges from about 50% to 60%), so that the importance of early detection is recognized.

Diagnostic imaging is a useful method for early detection of small renal cancer. However, when used for many subjects upon health examination, for example, diagnostic imaging cannot be said to be effective and the cost required for diagnosis is relatively high. Hence, it has been strongly desired to discover a blood marker that is specific to and has high sensitivity to renal cancer. It is thought that the use of such blood marker would enable relatively inexpensive high throughput examination or diagnosis.

Regarding detection or diagnosis of human renal cancer, International Publication No. WO2005/024603 discloses a method using differences in gene expression. Also, as a protein known to be increased in human renal cancer, in addition to MMP2 (Lein, M. et al., International Journal of Cancer, 2000, Vol. 85, pp. 801-804) thought to increase the mobility of cancer cells via degradation of extracellular matrices and TNFRSF7 (Nakatsuji, T., Clinical and Experimental Medicine, 2003, Vol. 2, pp. 192-196) the expression of which is known to be increased in cases of nephropathy, PDE8B (International Publication No. WO2004/042077), FLOT1 (International Publication No. WO2004/048933), CD5 (Nagase, Y. et al., International Journal of Urology, The Japanese Urological Association, 1991, Vol. 82, pp. 1781-1789), ECM1 (U.S. Patent No. 6303765), and the like are also known. It cannot be said that the specificity of these proteins is relatively high, so that methods for examining renal cancer with high sensitivity based on these expression levels alone have not been clinically used.

### DISCLOSURE OF THE INVENTION

### PROBLEM TO BE RESOLVED BY THE INVENTION

However, the above-mentioned known markers and marker candidates have poor specificity and/or sensitivity and efficient methods for detecting such markers from biological samples have not been established. Therefore, in general, these markers are not clinically used. Renal cancer markers with higher specificity and sensitivity are earnestly desired.

An object of the present invention is to provide a composition useful for diagnosis of a renal cancer and a method for detecting a renal cancer using the composition.

### MEANS FOR RESOLVING THE PROBLEM

Examples of a method for screening a marker include: a method for comparing renal cancer cells with non-cancer cells in terms of gene expression levels, protein expression levels, cell metabolite levels, or the like by some means; and a method for measuring the levels of genes, proteins, metabolites, or the like contained in body fluids of patients with or without renal cancer.

To resolve the above problem, the present inventors have now found a group of protein markers that are specifically detected in the blood plasma of renal cancer patients alone or a group of protein markers that are specifically detected in the blood plasma of healthy subjects alone.

The present invention has the following characteristics.
(1) A method for detecting a renal cancer *in vitro*, comprising measuring any one of or a plurality of polypeptides shown in SEQ ID NOS: 1 to 7 or fragments thereof in a biological sample from a subject.
(2) The method according to (1), wherein the fragment is a polypeptide fragment derived from the amino acid sequence of the polypeptide shown in any of SEQ ID NOS: 1 to 7, comprising the amino acid sequence shown in any of SEQ ID NOS: 8 to 16.
(3) The method according to (1) or (2), wherein the presence or amount of the polypeptide or fragment thereof is measured.
(4) The method according to any one of (1) to (3) above, wherein significant increase or decrease of the amount of the polypeptide or fragment thereof compared with a control sample is used as an indicator.
(5) The method according to any one of (1) to (4) above, wherein the polypeptide or fragment thereof is measured by an immunological method.
(6) The method according to any one of (1) to (5) above, wherein the measurement is performed using a substance capable of binding to the polypeptide or fragment thereof.
(7) The method according to (6) above, wherein the substance capable of binding is an antibody or fragment thereof.
(8) The method according to (7) above, wherein the antibody is labeled.
(9) The method according to any one of (1) to (8) above, comprising immunologically determining the presence or amount of one or more of the polypeptides or fragments thereof in the sample using an antibody or fragment thereof specifically binding to the polypeptides or fragments thereof and then using significant increase or decrease of the amount of the polypeptides or fragments thereof compared with a control sample as an indicator; or using the presence of the polypeptides or fragments thereof in either the sample or a control sample as an indicator, to detect the renal cancer.
(10) The method according to any one of (1) to (9) above, wherein the sample is blood, blood plasma, serum, or urine.
(11) The method according to any one of (1) to (9) above, wherein the sample is a kidney-derived tissue or cell.
(12) The method according to any one of (7) to (9) above, wherein the antibody is a monoclonal antibody or a polyclonal antibody.
(13) A composition for diagnosis or detection of a renal cancer, comprising one or more antibodies or fragments thereof or chemically modified derivatives thereof, which specifically bind to at least one of the polypeptides shown in SEQ ID NOS: 1 to 7 or fragments thereof.
(14) The composition according to (13) above, wherein the polypeptide fragment is a polypeptide fragment derived from the amino acid sequence of a polypeptide shown in any of SEQ ID NOS: 1 to 7, comprising the amino acid sequence shown in any of SEQ ID NOS: 8 to 16.
(15) The composition according to (13) or (14) above, wherein the polypeptide fragment comprises an epitope comprising at least 7 amino acids.
(16) The composition according to any one of (13) to (15) above, which is in the form of a kit.
(17) Use of the composition according to any one of (13) to (16) above for *in vitro* detection of a renal cancer in a subject.

Terms as used herein comprise definitions as described below.

The term "chemically modified derivative" as used herein refers to a derivative labeled with a label such as enzyme, fluorophore, or radioisotope, or a derivative having chemical modification such as acetylation, glycosylation, phosphorylation, or sulfation.

The term "composition for diagnosis or detection" as used herein refers to a composition that can be directly or indirectly used for: diagnosing or detecting the presence or absence of affection with renal cancer, the degree of affection, the presence or absence of improvement, or the degree of improvement; or screening for a candidate substance useful for prevention, improvement, or treatment of renal cancer.

The term "biological sample" used herein as a subject of detection or diagnosis refers to a sample that contains a target polypeptide, the expression level of which is increased or decreased along with the development of renal cancer, or a sample collected from a living body suspected of containing such target polypeptide.

The term "specifically binding to" as used herein means that an antibody or a fragment thereof forms an antigen-antibody complex with only a target polypeptide (that is, a renal cancer marker in the present invention), a mutant thereof, or a fragment thereof, but does not substantially form such complexes with other peptidic or polypeptidic substances. AS used herein, the term "substantially" means that non-specific formation of such complexes may take place, but to a minor extent.

The term "epitope" as used herein refers to a partial amino acid region (that is, an antigenic determinant) having antigenicity or immunogenicity in a target polypeptide of the present invention, a mutant thereof, or a fragment thereof. Such epitope generally comprises at least 5 amino acids, preferably at least 7 amino acids, or at least 8 amino acids, and more preferably at least 10 amino acids.

### ADVANTAGE OF THE INVENTION

The renal cancer markers of the present invention are found in a biological sample such as blood of a renal cancer patient, but are almost never or never found in the same of a healthy subject. Alternatively, the renal cancer markers are found in a biological sample alone of a healthy subject in contrast, but are almost never or never found in the same of a renal cancer patient. The simple use of the presence or amount of such markers as an indicator has a significant effect such that renal cancer can be easily detected using blood, for example.

### BRIEF DESCRIPTION OF THE DRAWINGS

Fig. 1 shows the MS peak intensity of the polypeptide shown in SEQ ID NO: 8 of the present invention.
Fig. 2 shows the MS peak intensities of the polypeptides shown in SEQ ID NOS: 9 to 12 in the present invention.
Fig. 3 shows MS peak intensities of the polypeptides shown in SEQ ID NOS: 13 to 16 of the present invention.
Fig. 4 shows the levels of ARHGAP25 peptide in blood plasmas of 4 renal cancer patients before and after extirpative surgery.

### BEST MODES FOR CARRYING OUT THE INVENTION

The present invention will be further described specifically as follows.

### <Renal cancer marker>

Renal cancer markers for *in vitro* detection of renal cancer using the composition of the present invention for diagnosis or detection of renal cancer are polypeptides shown in SEQ ID NOS: 1 to 7 or fragments thereof.

The polypeptides of SEQ ID NOS: 1 to 7 of the present invention are listed in Table 1 below with their gene names (GenBank registration names) and protein Nos. (Swissprot Accession Nos.), and their properties. Of these polypeptides, the polypeptide shown in SEQ ID NO: 1 was specifically detected in the blood plasmas of renal cancer patients, but not detected or detected at significantly lower levels in the blood plasma of healthy subjects compared with the blood plasma of renal cancer patients. Also the polypeptides shown in SEQ ID NOS: 2 to 7 were specifically detected in the blood plasma of healthy subjects, but not detected or detected at significantly lower levels in the blood plasma of renal cancer patients compared with the blood plasma of healthy subjects. In addition, the amino acid sequences or the nucleotide (or base) sequences of these polypeptides and/or genes (cDNAs) are available by accessing the data bank, such as NCBI, GenBank, or Swissprot.

**Table 1**

| SEQ ID NO: | Gene name | Protein No. | Properties |
|---|---|---|---|
| 1 | ARHGAP25 | P42331 | Rho GTPase-activating protein 25 |
| 2 | ZYX | Q15942 | Zyxin (Zyxin-2) |
| 3 | VASP | P50552 | Vasodilator-stimulated phosphoprotein (VASP) |
| 4 | RBP4 | P02753 | Plasma retinol-binding protein |
| 5 | SERPINF2 | P08697 | Alpha-2-antiplasmin |
| 6 | ARHGDIB | P52566 | Rho GDP-dissociation inhibitor 2 (Rho GDI 2) (Rho-GDI beta) (Ly-GDI) |
| 7 | NUCB1 | Q02818 | Nucleobindin-1 |

Also, each fragment of the above polypeptides of the present invention comprises at least 7, at least 8, at least 10, at least 15, preferably at least 20, at least 25, more preferably at least 30, at least 40, at least 50, at least 100, at least 200 continuous amino acid residues in the amino acid sequences of the polypeptides, and retains one or more epitopes. Such fragments are capable of immunospecifically binding to antibodies or fragments thereof of the present invention. When the above polypeptides are present in blood, for example, it is assumed that the polypeptides are present as a result of cleavage and fragmentation by an enzyme existing therein such as protease or peptidase.

Moreover, as described later in Example 1, in the present invention, the polypeptide shown in SEQ ID NO: 8 was detected at particularly higher levels in biological samples of renal cancer patients compared with healthy subjects. In contrast, the polypeptides shown in SEQ ID NOS: 9 to 16 were detected at particularly higher levels in biological samples of healthy subjects compared with renal cancer patients. These polypeptides are fragments of the polypeptides shown in SEQ ID NOS: 1, 2, and 5-7. Therefore, these polypeptides shown in SEQ ID NOS: 8-16 and fragments comprising each of them therewithin can be more preferably used as renal cancer markers. Table 2 below shows the polypeptides of SEQ ID NOS: 8-16 and their sites in the polypeptides shown in SEQ ID NOS: 1, 2, and 5-7.

Also, the amino acid sequences of SEQ ID NOS: 14 and 15 appear successively in the amino acid sequence of SEQ ID NO: 6. Therefore, a sequence formed by linking the amino acid sequences of SEQ ID NOS: 14 and 15; that is, a polypeptide represented by the sequence of from alanine at position 5 to lysine at position 30 in SEQ ID NO: 6, and fragments containing them therewithin are also more preferably used as renal cancer markers.

**Table 2**

| SEQ ID NO: | Sites in polypeptides shown in SEQ ID NOS: 1-7 |
|---|---|
| 8 | Methionine at position 1 to arginine at position 15 in SEQ ID NO: 1 |
| 9 | Valine at position 36 to arginine at position 54 in SEQ ID NO: 2 |
| 10 | Serine at position 185 to lysine at position 201 in SEQ ID NO: 2 |
| 11 | Glycine at position 254 to lysine at position 265 in SEQ ID NO: 2 |
| 12 | Serine at position 344 to lysine at position 354 in SEO ID NO: 2 |
| 13 | Leucine at position 476 to lysine at position 491 in SEQ ID NO: 5 |
| 14 | Alanine at position 5 to lysine at position 21 in SEQ ID NO: 6 |
| 15 | Leucine at position 22 to lysine at position 30 in SEQ ID NO: 6 |
| 16 | Leucine at position 452 to leucine at position 461 in SEQ ID NO: 7 |

All of the above target polypeptides for detection of a renal cancer in the present invention are **characterized in that** in renal cancer patients, the levels of the polypeptides in biological samples such as blood are significantly or remarkably higher or lower in subjects suffered from renal cancer than healthy subjects.

The polypeptides in the present invention can be prepared by a chemical synthesis method such as solid phase synthesis or a DNA recombination technique, which is a technique conventionally used in the art. The DNA recombination technique is preferably used in terms of the ease of procedures or purification.

First, polynucleotide sequences encoding partial sequences of the polypeptides of the present invention are chemically synthesized using an automatic DNA synthesizer. The phosphoramidite method is generally employed for such synthesis, which enables the automatic synthesis of a single-stranded DNA with a length of no more than approximately 100 nucleotides. The automatic DNA synthesizer is commercially available from, for example, Polygen or ABI.

With the use of the thus obtained polynucleotides as probes or primers, a cDNA clone of interest is obtained by known cDNA cloning; that is, by constructing a cDNA library via an RT-PCR method from poly A(+)RNA that is obtained by treating total RNA (which is extracted from a living tissue such as a renal tissue in which the above target gene is expressed) with an oligo dT cellulose column and then performing screening of the library, such as hybridization screening, expression screening, or antibody screening. If necessary, such cDNA clone can be further amplified by the PCR method. Therefore, cDNA corresponding to a gene of interest can be obtained.

Probes or primers are selected from a sequence of 15 to 100 continuous nucleotides based on the polypeptide sequences shown in SEQ ID NOS: 1-7 and then can be synthesized as described above. Also, cDNA cloning techniques are described in Sambrook J. and Russel, D., Molecular Cloning, A LABORATORY MANUAL, Cold Spring Harbor Laboratory Press, issued January 15, 2001, Vol. 1 7. 42-7. 45 and Vol. 2 8. 9-8. 17, for example.

Next, the above obtained cDNA clone is incorporated into an expression vector and then prokaryotic or eukaryotic host cells transformed or transfected with the vector are cultured, so that a polypeptide of interest can be obtained from the cells or culture supernatants. At this time, a nucleotide sequence encoding a secretory signal sequence is flanked at the 5' end of a DNA encoding a mature polypeptide of interest, so that the mature polypeptide can be secreted extracellularly.

Vectors and expression systems are available from Novagen, Takara Shuzo, Daiichi Pure Chemicals, Qiagen, Stratagene, Promega, Roche Diagnositics, Invitrogen, Genetics Institute, and Amersham Bioscience, for example. As host cells, prokaryotic cells such as bacteria (e.g., *Escherichia coli* and *Bacillus subtilis*), yeast (e.g., *Saccharomyces cerevisiae*), insect cells (e.g., Sf cells), mammalian cells (e.g., COS, CHO, BHK, and NIH3T3), and the like can be used. Vectors may contain, in addition to DNA encoding the polypeptide, regulatory elements such as a promoter (e.g., a lac promoter, a trp promoter, a P_{L} promoter, a P_{R} promoter, an SV40 viral promoter, a 3-phosphoglycerate kinase promoter, and a glycolytic enzyme promoter), an enhancer, a polyadenylation signal, a ribosomal binding site, a replication origin, a terminator, a selection marker (e.g., drug resistance genes such as an ampicillin resistance gene and a tetracycline resistance gene; and auxotrophic markers such as LEU2 and URA3), and the like.

Also, to facilitate purification of a polypeptide, an expression product can also be generated in the form of a fusion polypeptide wherein a peptidic label is bound to the C-terminus or the N-terminus of the polypeptide. Examples of a typical peptidic label include, but are not limited to, a histidine repeat (His tag) comprising 6 to 10 His residues, FLAG, a myc peptide, a GST polypeptide, and a GFP polypeptide.

When the polypeptides according to the present invention are produced without adding any peptidic label, the polypeptides can be purified and isolated by means known by persons skilled in the art using the physical or chemical nature. Examples of purification methods include one of or a combination of a plurality of techniques including ion exchange chromatography, gel filtration chromatography, normal phase chromatography, reverse phase chromatography, hydrophobic interaction chromatography, isoelectric point chromatography, high performance liquid chromatography (HPLC), electrophoresis, ammonium sulfate fractionation, salting-out, salting-in, ultrafiltration, and dialysis. Furthermore, when a peptidic label such as a histidine repeat, FLAG, myc, GST, or GFP is bound to the polypeptide, an example of a purification method is a method using affinity chromatography appropriate for each peptidic label that is generally used. In this case, an expression vector that makes isolation and purification easy is preferably constructed. In particular, an expression vector is constructed, so that a target polypeptide is expressed in the form of a fusion polypeptide of the polypeptide and a peptidic label. When the polypeptide is prepared by genetic engineering techniques, the isolation and purification thereof can be easily performed.

Examples of the above polypeptides in the present invention also include mutants thereof. The term "mutant" as used herein refers to: mutants comprising a deletion(s), substitution(s), addition(s), or insertion(s) of one or more, preferably one or several, amino acids in the amino acid sequences shown in SEQ ID NOS: 1-7 or partial sequences thereof; or mutants showing about 80% or more, about 85% or more, preferably about 90% or more, more preferably about 95% or more, about 97% or more, about 98% or more, or about 99% or more identity with the amino acid sequences or partial sequences thereof. Examples of such mutants include homologs of mammalian species differing from humans and natural mutants such as mutants based on polymorphic mutation among mammals of the same species (e.g., race).

The term "several" as used herein refers to an integer of 10, 9, 8, 7, 6, 5, 4, 3, or 2.

Also, the term "% identity" as used herein can be determined using a protein or nucleic acid homology searching program such as BLAST or FASTA with or without introduction of a gap (Karlin, S. et al., 1993, Proceedings of the National Academic Sciences U.S.A., Vol. 90, p. 5873-5877; Altschul, S.F. et al., 1990, Journal of Molecular Biology, Vol. 215, p. 403-410; Pearson, W.R. et al., 1988, Proceedings of the National Academic Sciences U.S.A., Vol. 85, p. 2444-2448; Ed., Toshihisa Takagi·Minoru Kanehisa, Method for use of GenomeNet database (2nd Version) 1998, KYORITSU SHUPPAN CO., LTD, Tokyo, Japan).

### <Composition for diagnosis or detection of renal cancer>

The present invention provides a composition for diagnosis or detection of a renal cancer, comprising one of or a plurality of antibodies or fragments thereof, chemically modified derivative thereof, which are capable of specifically binding to the polypeptides shown in SEQ ID NOS: 1-7 or fragments thereof.

Furthermore, more preferably, the composition for diagnosis or detection of a renal cancer in the present invention comprises one of or a plurality of antibodies or fragments thereof, or chemically modified derivative thereof, which are capable of specifically binding to fragments of the polypeptides shown in SEQ ID NOS: 1-7, each fragment comprising the amino acid sequence shown in any one of SEQ ID NOS: 8-16;.

An antibody that recognizes a polypeptide which is a renal cancer marker is capable of specifically binding to the polypeptide via an antigen binding site of the antibody. Such antibody usable in the present invention can be prepared by conventional techniques using polypeptides having the amino acid sequences of SEQ ID NOS: 1-7 or fragments thereof or using a fusion polypeptide(s) thereof as one or more immunogens. These polypeptides, fragments, mutants, or fusion polypeptides used as immunogens are: polypeptide fragments each comprising at least 7, at least 8, at least 10, at least 15, preferably at least 20, at least 25, at least 30, at least 40, at least 50, at least 100, or at least 200 continuous amino acid residues, derived from the amino acid sequence of each polypeptide shown in any one of SEQ ID NOS: 1-7; or each comprises the full-length polypeptide shown in any one of SEQ ID NOS: 1-7. Also, more preferably, these polypeptides, fragments, mutants, or fusion polypeptides used as immunogens are polypeptide fragments each comprising the amino acid sequence shown in any one of SEQ ID NOS: 8-16.

Examples of these polypeptides, or, fragments thereof, mutants, or fusion polypeptides include epitopes that induce antibody formation. These epitopes may be linear epitopes or discontinuous epitopes (higher order structures). In addition, such epitopes can be identified by all epitope analysis methods known in the art, such as a phage display method or a reverse immunogenetics method.

Examples of the antibodies in the present invention include, but are not limited to, antisera, polyclonal antibodies, monoclonal antibodies, chimeric antibodies, single-chain antibodies, humanized antibodies, and human antibodies.

Examples of antibodies that can be used in the present invention include antibodies of any types, classes, and subclasses. Examples of such antibodies include IgG, IgE, IgM, IgD, IgA, IgY, IgG1, IgG2, IgG3, IgG4, IgA1, and IgA2.

Moreover, antibodies in all forms are induced by the polypeptides according to the present invention. When the whole or part of the polypeptide or an epitope has been isolated, both polyclonal antibody and monoclonal antibody can be prepared using conventional techniques. An example of such method is as described in Monoclonal Antibodies, Hybridomas: A New Dimension in Biological Analyses, supervised by Kennet et al., Ple num Press, New York, 1980, for example.

A polyclonal antibody can be prepared by immunizing animals such as birds (e.g., chicken), mammals (e.g., a rabbit, a goat, a horse, sheep, and a mouse) with the polypeptide according to the present invention. The antibody of interest can be purified from the blood of immunized animals through an appropriate combination of techniques such as ammonium sulfate fractionation, ion exchange chromatography, and affinity chromatography.

A monoclonal antibody can be obtained by a technique that comprises producing a hybridoma cell line that produces a monoclonal antibody specific to each polypeptide in mice by a conventional technique. One method for producing such hybridoma cell line comprises immunizing animals with the polypeptide according to the present invention, collecting spleen cells from immunized animals, fusing the spleen cells to a myeloma cell line, so as to generate hybridoma cells, and then identifying the hybridoma cell line that produces a monoclonal antibody binding to the polypeptide. The monoclonal antibody can be collected by conventional techniques.

Preparation of monoclonal and polyclonal antibodies is as described in detail as follows.

### A. Preparation of monoclonal antibody

### (1) Immunization and collection of antibody-producing cell

An immunogen obtained as described above is administered to a mammal such as a rat, a mouse (e.g., the inbred mouse strain Balb/c), or a rabbit. The dose of the immunogen is appropriately determined depending on, for example, the type of an animal to be immunized or the route of administration, to be about 50 µg to 200 µg per animal. Immunization is primarily performed by injecting an immunogen subcutaneously or intraperitoneally. Also, the intervals of immunization are not particularly limited. After the primary immunization, boost immunization is carried out 2 to 10 times, and preferably 3 or 4 times, at intervals of several days to several weeks, and preferably at intervals of 1 to 4 weeks. After the primary immunization, the antibody titer of the blood serum of the immunized animal is repeatedly measured by, for example, ELISA (Enzyme-Linked Immuno Sorbent Assay). When the antibody titer reaches a plateau, the immunogen is injected intravenously or intraperitoneally to complete the final immunization. Antibody-producing cells are collected 2 to 5 days and preferably 3 days after the final immunization. Examples of antibody-producing cells include spleen cells, lymph node cells, and peripheral blood cells, and preferably spleen cells or regional lymph node cells.

### (2) Cell fusion

Hybridoma cell lines that produce monoclonal antibodies specific to each protein can be produced and then identified by conventional techniques. A method for producing such hybridoma cell lines comprises immunizing an animal with the polypeptide of the invention, removing spleen cells from the immunized animal, fusing the spleen cells to a myeloma cell line, producing hybridoma cells therefrom, and then identifying a hybridoma cell line that produces a monoclonal antibody binding to the enzyme of interest. Myeloma cell lines to be fused to antibody-producing cells, which can be used herein, are commercially available established cell lines of animals such as mice. Preferably, cell lines to be used herein have drug selectivity so that they cannot survive in a HAT selective medium (containing hypoxanthine, aminopterin, and thymidine) in an unfused state, but they can survive only in a state fused to antibody-producing cells. Such established cell lines are preferably derived from an animal of the same species with the immunized animal. A specific example of the myeloma cell line is a P3X63-Ag.8 strain (ATCC TIB9), which is a BALB/c mouse-derived hypoxanthine·guanine·phosphoribosyl·transferase (HGPRT) deficient cell line.

Subsequently, the myeloma cell lines are fused to the antibody-producing cells. Cell fusion is carried out in a serum-free medium for animal cell culture, such as DMEM or RPMI-1640 medium, by mixing the antibody-producing cells with the myeloma cell lines at about 1:1 to 20:1 1 in the presence of a cell fusion accelerator. As the cell fusion accelerator, polyethylene glycol or the like having an average molecular weight ranging from 1,500 to 4,000 daltons can be used at a concentration ranging from about 10% to 80%, for example. Optionally, an auxiliary agent, such as dimethyl sulfoxide, can be used in combination to enhance the fusion efficiency. Further, the antibody-producing cells can be fused to the myeloma cell lines using a commercially available cell fusion apparatus utilizing electric stimuli (e.g., electroporation).

### (3) Selection and cloning of hybridoma

The hybridomas of interest are selected from the fused cells. To this end, the cell suspension is adequately diluted with, for example, a fetal bovine serum-containing RPMI-1640 medium, then the suspension is aliquoted into each well of a microtiter plate at about two million cells/well, a selection medium is added to each well, and then culture is carried out while appropriately exchanging the selection medium with the same fresh medium. The culture temperature ranges from 20°C to 40°C and is preferably about 37°C. When the myeloma cell is an HGPRT-deficient cell line or thymidine kinase-deficient cell line, only a hybridoma of a cell having an ability to produce an antibody and a myeloma cell line can selectively be cultured and grown in the selection medium containing hypoxanthine, aminopterin, and thymidine (i.e., the HAT medium). As a result, cells that start to grow on about day 14 after the initiation of culture in the selection medium can be obtained as hybridoma cells.

Subsequently, whether or not the culture supernatant of the grown hybridomas contains the antibody of interest is screened for. Screening of hybridomas can be carried out in accordance with conventional techniques, without particular limitation. For example, the culture supernatant in a well containing the grown hybridomas is partially sampled and then subjected to enzyme immuno assay (EIA) or ELISA or radio immuno assay (RIA). The fused cells are cloned using the limiting dilution method or the like, and monoclonal antibody-producing cells, i.e. hybridomas, are established in the end. The hybridoma is stable during culture in a basic medium, such as RPMI-1640 or DMEM, and the hybridoma can produce and secrete a monoclonal antibody that reacts specifically with a polypeptidic cancer marker of the present invention.

### (4) Recovery of antibody

Monoclonal antibodies can be recovered by conventional techniques. Specifically, a monoclonal antibody can be collected from the established hybridoma by a conventional cell culture technique, ascites development, or the like. According to the cell culture technique, hybridomas are cultured in an animal cell culture medium, such as 10% fetal bovine serum-containing RPMI-1640 medium, MEM medium, or a serum-free medium, under common culture conditions (e.g., 37°C, 5% CO₂ concentration) for 2 to 10 days, and the antibody is obtained from the culture supernatant. In the case of ascites development, about 10 millions of hybridoma cells are administered intraperitoneally to an animal of the same species as the mammal from which the myeloma cells are derived, so as to allow the hybridoma cells to grow in large quantity. After one to two weeks, the ascites or blood serum is collected.

Where antibody purification is required in the above-described method for collecting the antibody, known techniques, such as salting out by ammonium sulfate, ion-exchange chromatography, affinity chromatography, and gel filtration chromatography, may be appropriately selected or combined to obtain the purified monoclonal antibody of the present invention.

### B. Preparation of polyclonal antibody

When polyclonal antibodies are prepared, an animal is immunized in the same manner as described above, the antibody titer is measured on days 6 to 60 after the final immunization by enzyme immunoassay (EIA or ELISA) or radio immunoassay (RIA), and blood is taken on the day at which the maximal antibody titer is measured, in order to obtain antiserum. Thereafter, the reactivity of the polyclonal antibodies in the antiserum is assayed by ELISA or the like.

Also, in the present invention, an antigen-binding fragment of the above antibodies can also be used. Examples of antigen-binding fragments that can be produced by conventional techniques include, but are not limited to, Fab and Fab', F(ab')₂, Fv, scFv, and dsFv. Examples thereof also include antibody fragments and derivatives thereof that can be produced by genetic engineering techniques. Examples of such antibodies include synthetic antibodies, recombinant antibodies, multi-specific antibodies (including bispecific antibodies), and single chain antibodies.

The antibodies of the present invention can be used *in vitro* and *in vivo.* In the present invention, the antibodies can be used for assays for detection of the presence of polypeptides or (poly)peptide fragments thereof. A monoclonal antibody is preferably used to enable specific detection in an assay. Even in the case of a polyclonal antibody, a specific antibody can be obtained by a so-called absorption method that comprises binding an antibody to an affinity column to which a purified polypeptide is bound.

Therefore, the composition of the present invention can contain at least one, preferably a plural number of types of, and more preferably all types of antibodies or fragments thereof capable of specifically binding to the polypeptides of SEQ ID NOS: 1-7, mutants thereof, or fragments thereof. Furthermore, more preferably, the composition of the present invention can contain at least one, preferably a plural number of types of, and more preferably all types of antibodies or fragments thereof capable of specifically binding to polypeptide fragments that are fragments of the polypeptides shown in SEQ ID NOS: 1-7 and each comprises the amino acid sequence shown in any one of SEQ ID NOS: 8-16.

In the case of the composition of the present invention, antibodies or fragments thereof capable of specifically binding to each of the above polypeptides can be individually packaged or appropriately mixed and then packaged as a mixture in individual containers (e.g., vials). Such antibodies or fragments thereof may be bound to a solid-phase support or in a free form. When bound to a solid-phase support, antibodies or fragments thereof may be adhered onto or bound onto a solid-phase support in the form of a multi-well plate, beads, a test tube, a stick, a specimen, or the like made of material such as polystyrene, polycarbonate, polyvinyl toluene, polypropylene, polyethylene, polyvinyl chloride, nylon, polymethacrylate, polymethylmethacrylate, polytetrafluoroethylene, poly(vinylidene fluoride), latex, agarose, cellulose, sepharose, glass, metal, ceramics, or magnetic material. In the case of free form, antibodies or fragments thereof are solids such as freeze-dried solids or liquids such as solutions.

A label, such as a fluorophore, an enzyme, or a radioisotope may be bound to an antibody or a fragment thereof to be used in the present invention, if necessary. Alternatively, such label may also be bound to a secondary antibody.

Examples of a fluorophore include fluorescein and a derivative thereof, rhodamine and a derivative thereof, dansyl chloride and a derivative thereof, and umbelliferone.

Examples of an enzyme include horseradish peroxidase, alkaline phosphatase, β-galactosidase, urease, catalase, glucose oxidase, lactate dehydrogenase, and amylase.

Examples of a radioisotope include iodines (¹³¹I, ¹²⁵I, ¹²³I, and ¹²¹I), phosphorus (³²P), sulfur (³⁵S), metals (e.g., ⁶⁸Ga, ⁶⁷Ga, ⁶⁸Ge, ⁵⁴Mn, ⁹⁹Mo, ⁹⁹Tc, and ¹³³Xe) and tritium.

Examples of other labels include luminescence substances such as NADH-, FMNH2-, acridinium ester, and luminol and bioluminescence substances such as luciferase and luciferin.

Also, if necessary, an avidin-biotin system or a streptavidin-biotin system can also be used herein. In this case, for example, biotin can be bound to the antibody or a fragment thereof of the present invention.

The composition of the present invention may further be in the form of a kit. The above antibodies for detection of renal cancer markers can be individually or appropriately mixed and then packaged in individual containers (e.g., vials). Furthermore, these antibodies may be bound to a solid-phase support or in a free form, as described above. The kit of the present invention may contain a labeled secondary antibody, a support, a washing buffer, a diluted sample, an enzyme substrate, a stop solution, a marker polypeptide as a purified standard substance, instructions, and the like.

### <Detection of renal cancer>

According to the present invention, renal cancer can be detected by a method that comprises examining the amount or the presence of the polypeptides shown in SEQ ID NOS: 1-7 or fragments thereof in a subject-derived biological sample, preferably one or a plurality of the polypeptides or fragments thereof, using substances capable of binding to the above renal cancer markers. It can be diagnosed by the method of the present invention that a subject has renal cancer when the polypeptide shown in SEQ ID NO: 1, a mutant(s), or a fragment(s) thereof is detected in a biological sample of the subject or the expression level of such polypeptide, mutant(s), or fragment(s) thereof is determined to be significantly higher than a control level, or when the polypeptides shown in SEQ ID NOS: 2-7, mutants thereof, or fragments thereof are not detected or the expression levels of such polypeptides, mutants, or fragments thereof are determined to be significantly below control expression levels.

Also, more preferably, renal cancer can be detected by a method that comprises examining the amount or the presence of one or a plurality of polypeptide fragments that are fragments of the polypeptides shown in SEQ ID NOS: 1-7 and each comprises the amino acid sequence shown in any one of SEQ ID NOS: 8-16. It can be diagnosed or determined by the method of the present invention that a subject has renal cancer when a polypeptide fragment comprising the amino acid sequence shown in SEQ ID NO: 8 is detected in a biological sample of the subject or the expression level of the polypeptide, mutant thereof, or fragment thereof is determined to be significantly higher than the control level, or when polypeptide fragments comprising the amino acid sequences shown in SEQ ID NOS: 9-16 are not detected or the expression levels of such polypeptides, mutants thereof, or fragments thereof are determined to be significantly lower than the control levels.

In the method of the present invention, the above detection of renal cancer markers may be performed using a single marker, but is preferably performed using a plurality of (e.g., 2 or more, 3 or more, 4 or more, or 5 or more to 14, 15, or 16 or less) markers. This is intended to avoid unpredictable detection of a non-specific complex, in other words, misdiagnosis. Also, the renal cancer markers in the present invention may be used in combination with other cancer diagnostic markers known by persons skilled in the art.

The composition of the present invention is useful for diagnosis, determination, or detection of renal cancer; that is, diagnosis of the presence or the absence of the disease or the degree of the disease (how the subject is affected). In diagnosis of renal cancer, comparison is made with controls such as normal tissues (or cells), non-cancer tissues (or cells), and normal body fluids, and then the presence or the amount of the above renal cancer markers in a biological sample of the subject is detected. When differences in terms of the presence or the amount are found to be significant, the subject is suspected of having renal cancer.

Examples of test samples to be used in the present invention include renal tissues, peripheral tissues thereof, renal cells, or body fluids such as blood, serum, blood plasma, a renal fluid, and urine. Body tissues of a subject are collected by biopsy or the like or obtained by surgery.

Subjects in the present invention are mammals including humans and are preferably humans.

Examples of the above substances capable of binding to renal cancer markers include the above antibodies or fragments thereof, aptamers, Affibody (Affibody Trade Mark), receptors of these renal cancer markers, substances inhibiting the specific action of these renal cancer markers, and substances activating specific action of these renal cancer markers. Examples thereof are preferably antibodies or fragments thereof or chemically modified derivatives thereof.

In an embodiment of the present invention, measurement can comprise the steps of: bringing an antibody or a fragment thereof labeled with (if necessary) a common enzyme or fluorophore into contact with a tissue section or a homogenized tissue or body fluid; and qualitatively or quantitatively measuring an antigen-antibody complex. Detection is carried out by, for example, a method for measuring the presence and the level of a target polypeptide by immunoelectron microscopy, or a method for measuring the presence or the levels of target polypeptides by a conventional method such as an enzyme antibody method (e.g., ELISA), a fluorescent antibody technique, a radioimmunoassay, a homogeneous method, a heterogeneous method, a solid phase method, and a sandwich method. Furthermore, in the present invention, for easy detection of a reaction between the antibody of the present invention and a target polypeptide in a sample, the reaction is directly detected by labeling the antibody of the present invention or indirectly detected using a labeled secondary antibody. In the case of the detection method of the present invention, the latter indirect detection is preferably used in terms of sensitivity.

It is determined that a subject has renal cancer when, depending on types of the above renal cancer markers: a target polypeptide is found to be present or disappear in a body fluid or a renal cancer tissue or cells or preferably blood obtained from the subject; or the level of the target polypeptide is found to be significantly increased or decreased compared with the control level. As used herein, the term "significantly" refers to the presence of a statistically significant difference, wherein the significance level is less than 0.05.

An example of a measurement method as an alternative for an immunological method is a method using mass spectrometry. This method can be performed specifically by techniques described in the Examples. Specifically, a biological sample such as serum or blood plasma is filtered using a filter to remove contaminants, diluted with a buffer (e.g., pH, about 8), and then adjusted to have a concentration ranging from approximately 10 mg/ml to approximately 15 mg/ml. Subsequently, the resultant is filtered using a hollow fiber filter (Reference example (1) below) or a centrifugal flat membrane filter, which is capable of removing proteins with a molecular weight of 50,000 or more, so as to perform molecular weight fractionation. The fractions are treated with protease (e.g., trypsin) for peptidization and then the resultants are subjected to a mass spectrometer (the type using matrix-assisted laser desorption ionization or electrospray ionization). Differences between the amount of a polypeptide existing in a sample of a patient (before surgery to remove renal cancer) and the same of a healthy subject can be measured based on the mass to charge ratio and intensity at a specific peak from the polypeptide of interest.

### EXAMPLES

The present invention will be described in more detail with reference to the examples set forth below; however, the technical scope of the present invention is not limited to the examples.

### <Reference example>

### (1) Preparation of hollow fiber filter

A hundred polysulfone hollow fibers having a pore size (molecular weight cut off) of approximately 50,000 on the membrane surface were packed into a bundle. The both ends of the bundle were fixed to a glass tube using an epoxy-based potting agent so as not to block the hollow parts of the hollow fibers, so that a mini module is prepared. The mini module (module A) was used for removal of high-molecular-weight proteins in serum or blood plasma, having a diameter of approximately 7 mm and a length of approximately 17 cm. Similarly, a mini module (module B) to be used for condensing low-molecular-weight proteins was prepared using a membrane with a pore size (molecular weight cut off) of approximately 3,000. A mini module has an inlet that is connected to hollow fiber lumen on one end and an outlet on the other end. The inlets and outlets of hollow fibers are closed circulatory system passages in the form of a silicon tube. Through the passages, a liquid is driven by a. Peristar pump to circulate. Also, a glass tube of the hollow fiber mantle is provided with a port for discharging a liquid leaking from the hollow fibers, so that one module set is constituted. A module was connected to a position in the middle of such passage using a "T"-shaped connector, three modules A and one module B1 were connected in tandem, thereby forming one hollow fiber filter. The hollow fiber filter was washed with distilled water, and then filled with an aqueous PBS solution (phosphate buffer containing 0.15 mM NaCl, pH 7.4). A fraction raw material, serum or blood plasma, was injected from the passage inlet of the hollow fiber filter and then discharged from the passage outlet after fractionation and condensation. Serum or blood plasma injected to the hollow fiber filter was applied to a molecular sieve with a molecular weight cut off of approximately 50,000 for every module A. Thus, components with molecular weights lower than that of 50,000 are condensed using the module B and then prepared.

### <Example 1>

### (1) Identification of proteins in the blood plasma of healthy subjects and renal cancer patients

EDTA blood plasma was obtained from seven renal cancer patients in their 50s to 70s before extirpative surgery and at 1 month after extirpative surgery. EDTA blood plasma was also obtained from eight healthy subjects of similar age. Measurement was carried out for the blood plasma. Blood plasma was filtered using a filter having a pore size of 0.22 µm to remove contaminants and then the protein concentration was adjusted to 50 mg/mL. The resulting blood plasma was further diluted in a 25 mM ammonium bicarbonate solution (pH 8.0) to the concentration of 12.5 mg/ml, and molecular weight fractionation was then carried out using the hollow fiber filter as described in Reference example (1). Each fractionated blood plasma sample (total amount of 1.8 ml, comprising 250 µg (max) of proteins) was divided into 7 fractions by reversed-phase chromatography (the ProteomeLab^{®} PF2D System (Beckman Coulter)). The fractions were each lyophilized and then redissolved in 100 µL of the 25 mM ammonium bicarbonate solution (pH 8.0). This sample was digested with trypsin (in a volume 1/50 the total protein) at 37°C for 2 to 3 hours for peptidization. Each peptide fraction was further fractionated into 4 fractions on the ion-exchange column (KYA Technologies, Japan). Each of the resulting fractions was further fractionated on a reversed phase column (KYA Technologies, Japan). The thus eluted peptides were measured using a mass spectrometer Q-TOF Ultima (Micromass) connected online at a survey scan mode.

Generally in the art, the ratio of mass to electric charge is corrected using a' standard substance upon measurement. In addition to this, several proteins that are generally always detected as blood proteins (e.g., albumin and α-fibrinogen) were used as internal standards in analysis of the present invention. Measurement data were re-corrected using theoretical masses of these proteins, so as to further refine the data. The thus refined data were analyzed using protein analysis software MASCOT (Matrix Science, England). The two following standards were used for identification of blood proteins: (i) among peptides belonging to a protein, at least one peptide is detected with reliability as high as a 34 or greater Mowse score; and (ii) the difference (error) between the measured value of the MS data and the measured value of MS/MS data of a peptide and the theoretical value of the peptide is 0.03 daltons or less. Thus, analysis was carried out under conditions where false protein identification can be eliminated as far as possible.

### (2) Proteins in blood plasma (before surgery to remove cancer) detected in increased or decreased number of renal cancer patients, compared with healthy subjects

The data were compared between healthy subjects and cancer patients. Of the thus identified proteins, proteins detected in the blood plasma (before surgery to remove cancer) of 3 or more renal cancer patients, but never detected in the same of healthy subjects, were found as proteins whose expression was significantly enhanced in the blood plasma of patients (before surgery to remove renal cancer) compared with healthy subjects. These proteins correspond to the polypeptide shown in SEQ ID NO: 1 listed in Table 3 below. Also, from among the thus identified proteins, a protein that was detected in the blood plasma of healthy subjects greater in number by 3 or more than those in renal cancer patients (before surgery) was found as a protein whose expression level was significantly decreased in the blood plasma of a patient (before surgery to remove renal cancer) than in a healthy subject. These proteins are polypeptides shown in SEQ ID NOS: 2-7 listed in Table 3 below. Therefore, it was demonstrated that these polypeptides shown in SEQ ID NOS: 1-7 are useful as renal cancer markers for detection of renal cancer.

**Table 3**

| SEQ ID NO: | Gene Name | Number of subjects of each group from which proteins were detected | |
|---|---|---|---|
| | | Healthy subject | Patient before surgery to remove renal cancer |
| 1 | ARHGAP25 | 0 | 4 |
| 2 | ZYX | 7 | 1 |
| 3 | VASP | 6 | 1 |
| 4 | RBP4 | 5 | 0 |
| 5 | SERPINF2 | 5 | 0 |
| 6 | ARHGDIB | 5 | 1 |
| 7 | NUCB1 | 3 | 0 |

### (3) Proteins for which MS peak intensity was increased or decreased in the blood plasma (before surgery to remove cancer) of renal cancer patients compared with healthy subjects

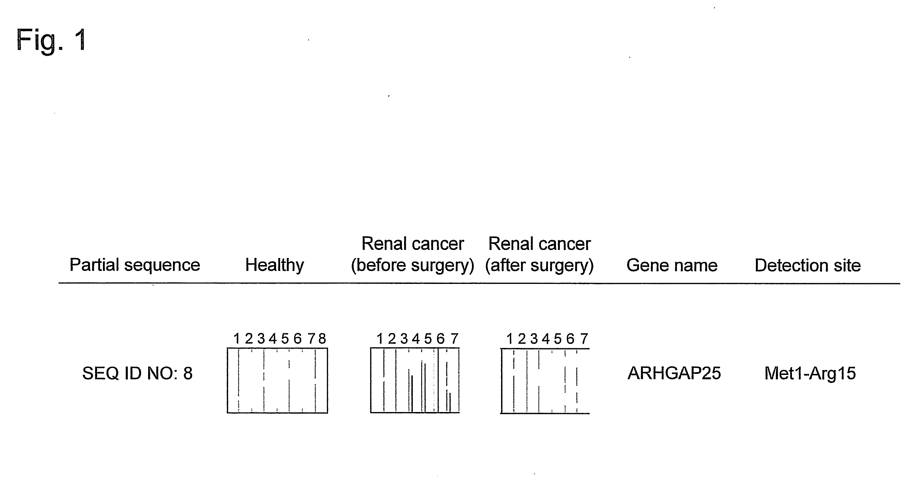

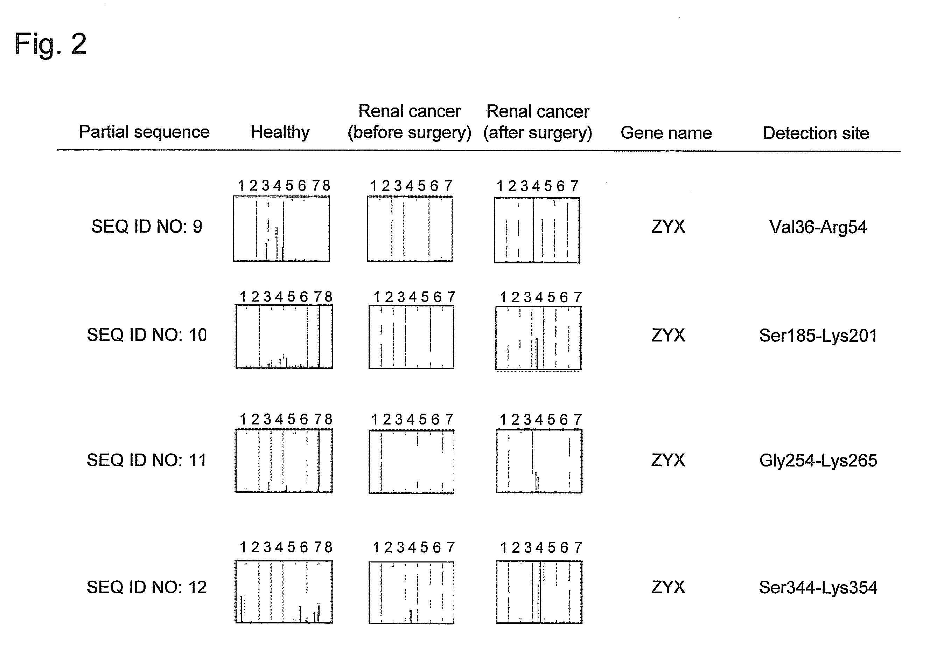

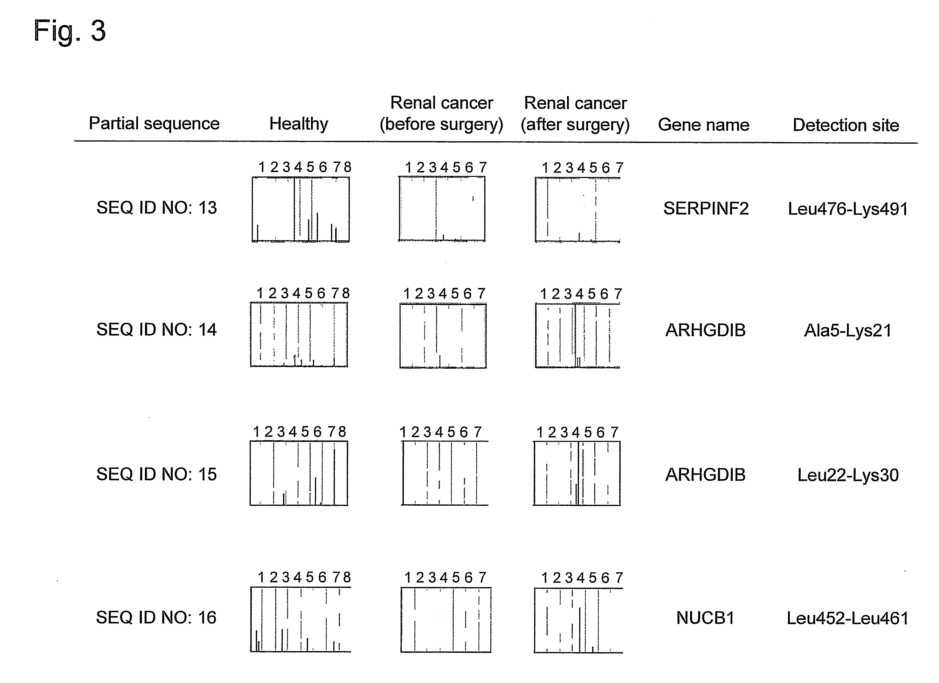

Furthermore, MS peak intensities at the time of detection of peptides belonging to the proteins identified in (2) above were analyzed. Such MS peak intensity is not strictly quantitative, but is a numerical value somewhat reflecting the amount of a protein existing therein. The fact that quantitative and comparative analysis using MS peak intensity is possible is known by persons skilled in the art. MS peak intensity at the time of each peptide detection was compared between healthy subjects and cancer patients. Among the thus detected peptides, the following peptides were detected as peptides that were found to significantly increase in amount in the blood plasma of patients (before surgery to remove renal cancer). The detected peptides had peak intensities in the blood plasma (before surgery to remove cancer) of renal cancer patients significantly higher than healthy subjects and also higher than those found in blood plasma at 1 month after surgery to remove cancer. These peptides correspond to the polypeptide shown in SEQ ID NO: 8 and are fragments of the polypeptide shown in SEQ ID NO: 1. Also similarly, among the thus detected peptides, the following peptides were detected as peptides whose amounts were found to significantly decrease in the blood plasma of patients (before surgery to remove renal cancer) compared with healthy subjects: that is, peptides, for which the peak intensities in the blood plasma (before surgery to remove cancer) of renal cancer patients were significantly lower than those of healthy subjects, and in addition the peak intensities in the blood plasma (before surgery to remove cancer) of renal cancer patients were lower than the peak intensities in the blood plasma at 1 month after surgery to remove cancer. These peptides are the polypeptides shown in SEQ ID NOS: 9-16. Each of these peptides is a fragment of the polypeptides shown in SEQ ID NOS: 2 and 5-7. Therefore, it was demonstrated that these polypeptides shown in SEQ ID NOS: 8-16 and fragments comprising each of them are useful as renal cancer markers for detection of renal cancer. Table 4 shows gene names of these polypeptide fragments shown in SEQ ID NOS: 8-16 and sites at which fragments are located in the sequences shown in SEQ ID NOS: 1, 2, and 5-7, in addition to peptide lengths and amino acid sequences (one-letter notation). Also, Figs. 1-3 show the MS peak intensities of the polypeptide fragments shown in SEQ ID NOS: 8-16 in healthy subjects, renal cancer patients (before surgery), and renal cancer patients (after surgery). Numerical figures above the MS peak intensities of renal cancer patients and healthy subjects of each sequence denote numbers of individual subjects.

**Table 4**

| SEQ ID NO: | Original sequence | Gene name | Relevant part of the full-length sequence | Peptide length (number of residues) | Peptide sequence (one-letter notation) |
|---|---|---|---|---|---|
| 8 | SEQ ID NO: 1 | ARHGAP25 | 1-15 | 15 | MSLGQSACLFLSIAR |
| 9 | SEQ ID NO: 2 | ZYX | 36-54 | 19 | VNPFRPGDSEPPPAPGAQR |
| 10 | SEQ ID NO: 2 | ZYX | 185 - 201 | 17 | SSTKPAAGGTAPLPPWK |
| 11 | SEQ ID NO: 2 | ZYX | 254 - 265 | 12 | GPPASSPAPAPK |
| 12 | SEQ ID NO: 2 | ZYX | 344 - 354 | 11 | SPGAPGPLTLK |
| 13 | SEQ ID NO: 5 | SERPINF2 | 476 - 491 | 16 | LVPPMEEDYPQFGSPK |
| 14 | SEQ ID NO: 6 | ARHGDIB | 5 - 21 | 17 | APEPHVEEDDDDELDSK |
| 15 | SEQ ID NO: 6 | ARHGDIB | 22 - 30 | 9 | LNYKPPPQK |
| 16 | SEQ ID NO: 7 | NUCB1 | 452 - 461 | 10 | LPEVEVPQHL |

### <Example 2>

### (1) Preparation of human ARHGAP25

One of the renal cancer markers in the present invention is the human ARHGAP25 polypeptide shown in SEQ ID NO: 1. Also, as shown in Example 1 (3) and Fig. 1, a site in the human ARHGAP25 polypeptide, which is further characteristically detected in a renal cancer patient, is a sequence (hereinafter, ARHGAP25 peptide) of from the 1^{st} amino acid to the 15^{th} amino acid of the human ARHGAP25 polypeptide shown in SEQ ID NO: 8. The ARHGAP25 peptide could be chemically synthesized through commission at Takara Bio Inc. (Japan). Also, a rabbit polyclonal antibody against the ARHGAP25 peptide was obtained (prepared) through commission on Takara Bio Inc. Moreover, as an immunogen for obtainment of a mouse monoclonal antibody for detection of the ARHGAP25 peptide, the polypeptide (hereinafter, ARHGAP25 (1-97)) shown in SEQ ID NO: 17 comprising a region between the 1st amino acid and the 97^{th} amino acid comprising the amino acid sequence shown in SEQ ID NO: 8 in the amino acid sequence shown in SEQ ID NO: 1 was prepared as a recombinant protein from *Escherichia coli.* Procedures for preparation of ARHGAP25 (1-97) are as described below.

First, human ARHGAP25 mRNA was prepared from HEK293 cells. The mRNA was prepared specifically according to the attached protocols using a Qia shredder and an RNeasy mini kit (Qiagen). Next, cDNA was synthesized using reverse transcriptase SuperscriptII (Invitrogen) and the thus obtained total mRNA as a template and then a human cDNA library was constructed. Reverse transcription reaction was carried out according to the protocols attached to the above enzyme. Subsequently, PCR was carried out using the thus obtained human cDNA library as a template and a set of primers having the nucleotide sequences shown in SEQ ID NOS: 18 and 19. The nucleotide sequence shown in SEQ ID NO: 18 comprises a portion of the ARHGAP25 gene and a *Bam*H I recognition sequence upstream of the portion. The nucleotide sequence shown in SEQ ID NO: 19 comprises a portion of the ARHGAP25 gene and an *Eco*R I recognition sequence downstream of the portion. When gene amplification was carried out by the PCR method using these primers, a DNA sequence encoding ARHGAP25 (1-97) was obtained as an amplified DNA fragment. The PCR reaction was carried out using KOD (Toyobo Co., Ltd., Japan) as DNA polymerase and then preparation was carried out in accordance with protocols attached to the KOD so that the resultant contained 10 ng of the cDNA library and 10 pmol of each primer. Reaction was carried out under conditions of 1 minute of heating at a temperature of 94°C, 30 times of repetition of a reaction cycle of 94°C for 30 seconds, 55°C for 30 seconds, keeping the temperature at 72°C for 1 minute, and finally keeping the temperature at 72°C for 4 minutes. The thus amplified DNA fragment was purified using Quantum prep PCR Kleen Spin Columns (Bio-rad). A PCR product with a full length of approximately 300 bp was obtained by the reaction.

The thus obtained DNA fragment was cleaved with *Bam*H I and *Eco*R I, and at the same time the terminal portions were dephosphorylated using a BAP enzyme. Furthermore, purification was carried out by an agarose gel extraction method. The DNA fragment was mixed with a histidine tag fusion expression vector pET30b (Novagen) cleaved in advance with *Bam*H I and *Eco*R I, and then a ligation reaction was carried out. Ligation High (Toyobo Co., Ltd., Japan) was used as DNA ligase and the reaction was carried out according to attached protocols. Subsequently, competent cells were transformed using the solution obtained after ligation reaction. Competent cells used herein were cells of *Escherichia coli* DH5α strain (Takara Bio Inc., Japan). Transformation was specifically carried out according to the attached protocols. Transformed cells were applied onto an LB plate containing 100 µg/mL antibiotic ampicillin and then cultured overnight at 37°C. The thus obtained transformant was cultured overnight at 37°C in an LB liquid medium containing 100 µg/mL ampicillin, so that target pET30b_ARHGAP25 (1-97) was obtained by mini-prep.

For preparation of recombinant ARHGAP25 (1-97), *Escherichia coli* Rosetta-Gami 2 strain (Novagen) was transformed with pET30b_ARHGAP25 (1-97). The thus obtained transformant was cultured at 37°C for 3 hours using 3 L of an LB medium containing ampicillin and chloramphenicol. IPTG with a final concentration of 1 mM was added and then the transformant was cultured at 30°C for 18 hours. After the expression of target histidine tag fusion recombinant ARHGAP25 (1-97) was induced, cells were collected by centrifugation.

After the thus obtained cells were washed with PBS, insoluble fractions were prepared as precipitates using B-PER (PIERCE). Preparation was specifically carried out according to the attached protocols. Next, the insoluble fractions were solubilized using PBS containing 8M Urea and then the histidine tag fusion ARHGAP25 was adsorbed using Ni-NTA agarose resin (QIAGEN). The resin to which the protein had been adsorbed was washed with PBS containing 10 mM imidazole and then elution was carried out using a 1M imidazole solution. Next, from the thus obtained elution fraction, protein refolding was carried out. First, overnight dialysis in a PBS-T solution containing 6 M guanidine hydrochloride and then overnight dialysis in a PBS-T solution containing 0.4 M arginine and 1 mM DTT were carried out. The thus obtained refolding solution was subjected to acrylamide gel electrophoresis, and then purification of ARHGAP25 (1-97) was confirmed by Coomassie brilliant blue staining.

### (2) Preparation of mouse monoclonal antibody against human ARHGAP25 peptide

The 2 mg/mL human ARHGAP25 (1-97) solution (100 µL) obtained in (1) above was mixed with 100 µL of MPL+TDM Emulsion (Corixa). The total amount of the resultant was administered intraperitoneally to a 7-week-old BALB/c mouse. At week 2 and week 4 after administration, the same amount of a similarly prepared ARHGAP25 (1-97) solution was administered. Five weeks later, 100 µL of blood was collected via mouse tail vein, left to stand overnight, and then centrifuged at 5000 x g for 5 minutes, so that the supernatant was recovered as a partial serum. With the use of the serum, the antibody titer against the ARHGAP25 peptide was evaluated.

Six weeks later, an ARHGAP25 (1-97) solution prepared similarly to the above was administered intraperitoneally to a mouse for which production of the antibody against the ARHGAP25 peptide had been confirmed. Three days later, splenectomy was performed. The thus extracted spleen was perforated with a syringe and then an RPMI1640 medium (GIBCO) was injected to extrude spleen cells, so that a spleen cell solution was obtained. The thus obtained spleen cell solution was centrifuged at 1200 rpm for 7 minutes to remove the supernatant and then the resultant was washed with an RPMI1640 medium. The resultant was suspended again in an RPMI1640 medium and then the number of cells was counted, so that a solution of SP2/0 myeloma cells 1/10 the number of spleen cells was prepared. Both cell solutions were mixed, the mixture was centrifuged at 2200 rpm for 10 minutes, and then the supernatant was discarded. Cells were loosen by tapping. One (1) mL of a solution prepared by mixing PEG (ROCHE) with HBSS (GIBCO) at 5 : 1 was added to the cells and then the mixture was stirred. In the following procedures, all solutions or media used were kept at 37°C, unless particularly specified..

Nine (9) mL of an RPMI1640 medium was added for 5 minutes to and slowly mixed with the cell solution supplemented with PEG and HBSS, followed by 10 minutes of centrifugation at 2200 rpm. Thus, the supernatant was removed. The thus obtained precipitated cells were suspended in an RPMI1640 medium supplemented with 15% FCS and HAT (ROCHE). The suspension was injected to a 96-well cell culture plate (Greiner Bio-One) at 200 µL per well, followed by 1 week of culture at 37°C under 5% CO₂.

Colonies that had grown under HAT-supplemented conditions were determined to be hybridomas resulting from the fusion of spleen cells to myeloma cells. These hybridomas were subjected to selection of hybridomas producing an antibody against the ARHGAP25 peptide. First, 1 µg/mL ARHGAP25 peptide was added to at 50 µL per well of a 96-well peptide adsorption plate (Nunc) and then immobilized overnight. The protein solutions in wells were discarded, 200 µL of a BlockAce solution (Dainippon Sumitomo Pharma Co., Ltd., Japan) diluted 4-fold was added, and then the plate was left to stand for 1 hour at room temperature. Subsequently, the plate was washed with PBS-T, so that an ARHGAP25 peptide-immobilized plate was prepared. The supernatant in each well in which colonies had grown was diluted 5-fold, added to the ARHGAP25 peptide-immobilized plate at 100 µL per well, and then left to stand at room temperature for 1 hour. Subsequently, solutions in wells were discarded and then the plate was washed with PBS-T. An HRP-labeled anti-mouse IgG solution (Dako) (100 µL) was added and then the plate was left to stand at room temperature for 1 hour. The solutions in wells were discarded and then the plate was washed with PBS-T. A TMB solution (100 µL) was added so that a reaction was carried out for 15 minutes. Color developed by the reaction was confirmed with absorbance at 450 nm. Wells confirmed to show color development were determined to be positive. Colonies in positive wells were suspended in RPMI media containing 15% FCS and HT (invitrogen), so that cloning of positive clones was carried out by limiting dilution. Twenty (20) types of hybridomas obtained as a result of cloning were acclimatized in SFM media, so that antibody production was performed. The hybridomas were seeded on 60 mL of 100% SFM medium at 1 x 10⁵ cells/mL and then cultured for 10 days until cell death. Each culture solution was centrifuged at 3000 rpm for 15 minutes, so that cells were removed. The thus obtained culture supernatants were subjected to purification of antibodies contained therein using a MabTrap Kit (GE HEALTHCARE BIOSCIENCE, Japan). Also, 20 types of antibody were each subjected to biotinylation using a biotinylation reagent Sulfo-NHS-Biotin (PIERCE). Biotinylation was carried out according to protocols attached to the reagent.

### (3) Detection of human ARHGAP25 peptide in blood plasma of renal cancer patients by sandwich ELISA method using obtained antibodies

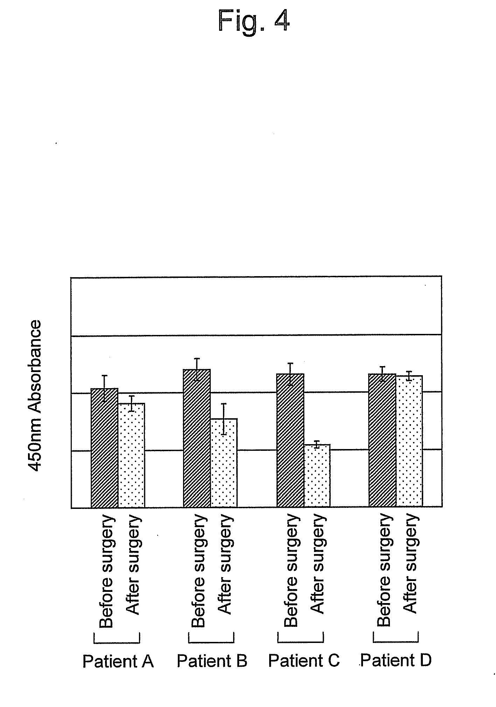

With the use of purified antibodies and biotin-labeled purified antibodies obtained in (2) above, examination was performed for construction of a detection system using a sandwich ELISA method. An unbiotinylated purified antibody was used as a capturing antibody and a biotin-labeled purified antibody was used as an antibody for detection. Thus combinations each consisting of a type of capturing antibody and a type of antibody for detection, capable of detecting the human ARHGAP25 peptide with the highest sensitivity, were selected. The capturing antibody was prepared in the form of a 2 µg/mL PBS solution and then added at 50 µL per well of a 96-well protein adsorption plate (Nunc), followed by overnight immobilization. On the next day, blocking was performed at room temperature for 2 hours using a PBS-T solution containing 1% BSA and 10% sucrose. Next, as shown in Example 1 and Fig. 1, the ARHGAP25 peptide was strongly detected in 4 blood plasma specimens from patients (before surgery to remove renal cancer) and 4 postoperative blood plasma specimens. Each of these 8 blood plasma specimens in total was diluted 2-fold using 1% BSA-PBST and then 100 µL each thereof was added to each well of the antibody-immobilized plate, followed by 2 hours of reaction at room temperature. After reaction, sample solutions within wells were discarded and then the plate was washed with PBS-T, followed by 1 hour of reaction at room temperature with 50 µL of 200 ng/mL antibody for detection diluted with 1% BSA-PBST. After reaction, wells were washed and then a reaction was performed at room temperature for 30 minutes with 100 µL of an avidin-HRP solution (R&D). Avidin-HRP was diluted with PBS. After washing with PBS-T, 100 µL of a TMB solution (PIERCE) was added. After 15 minutes of reaction, a 1 N sulfuric acid solution (100 µL) was added to stop the reaction and then absorbance at 450 nm was measured. As a result, as shown in Fig. 4, in all renal cancer patients, the concentration of the ARHGAP25 peptide in blood plasma obtained before extirpative surgery was found to be higher than the same obtained after extirpative surgery. Also, in blood plasma of 2 out of 4 renal cancer patients, the concentration of the ARHGAP25 peptide before extirpative surgery was found to be significantly higher than the same after surgery. Therefore, it was revealed that the ARHGAP25 peptide and the ARHGAP25 protein containing the same shown in Example 1 are useful as markers to determine whether or not a subject is affected with renal cancer.

### INDUSTRIAL APPLICABILITY

According to the present invention, compositions with good specificity and sensitivity for diagnosis and detection of renal cancer can be provided. The present invention is particularly useful in the field of medicine.

This description includes all or part of the contents as disclosed in the description of Japanese Patent Application No. 2008-008411, which is a priority document of the present application. Also, all publications, patents, and patent applications cited herein are incorporated herein by reference in their entirety.

## Claims

1. A method for detecting a renal cancer *in vitro*, comprising measuring any one of or a plurality of polypeptides shown in SEQ ID NOS: 1 to 7 or fragments thereof in a biological sample from a subject.

2. The method according to claim 1, wherein the fragment is a polypeptide fragment derived from the amino acid sequence of the polypeptide shown in any of SEQ ID NOS: 1 to 7, comprising the amino acid sequence shown in any of SEQ ID NOS: 8 to 16.

3. The method according to claim 1, wherein the presence or amount of the polypeptide or fragment thereof is measured.

4. The method according to claim 3, wherein significant increase or decrease of the amount of the polypeptide or fragment thereof compared with a control sample is used as an indicator.

5. The method according to claim 1, wherein the polypeptide or fragment thereof is measured by an immunological method.

6. The method according to claim 1, wherein the measurement is performed using a substance capable of binding to the polypeptide or fragment thereof.

7. The method according to claim 6, wherein the substance capable of binding is an antibody or fragment thereof.

8. The method according to claim 7, wherein the antibody is labeled.

9. The method according to claim 1, comprising immunologically determining the presence or amount of one or more of the polypeptides or fragments thereof in the sample using an antibody or fragment thereof specifically binding to the polypeptides or fragments thereof and then using significant increase or decrease of the amount of the polypeptides or fragments thereof compared with a control sample as an indicator; or using the presence of the polypeptides or fragments thereof in either the sample or a control sample as an indicator, to detect the renal cancer.

10. The method according to claim 1, wherein the sample is blood, blood plasma, serum or urine, or a kidney-derived tissue or cell.

11. The method according to claim 7, wherein the antibody is a monoclonal antibody or a polyclonal antibody.

12. A composition for diagnosis or detection of a renal cancer, comprising one or more antibodies or fragments thereof or chemically modified derivatives thereof, which specifically bind to at least one of the polypeptides shown in SEQ ID NOS: 1 to 7 or fragments thereof.

13. The composition according to claim 12, wherein the polypeptide fragment is a polypeptide fragment derived from the amino acid sequence of a polypeptide shown in any of SEQ ID NOS: 1 to 7, comprising the amino acid sequence shown in any of SEQ ID NOS: 8 to 16.

14. The composition according to claim 12, wherein the polypeptide fragment comprises an epitope comprising at least 7 amino acids.

15. The composition according to claim 12, which is in the form of a kit.

16. Use of the composition according to claim 12 for *in vitro* detection of a renal cancer in a subj ect.


SEQUENCE LISTING

<110> Toray Industries, Inc.

<120> Composition and method for diagnosis or detection of renal
      cancer

<130> PH-3815-PCT

<140> PCT/JP2009/05024
<141> 2009-01-16

<150> JP 2008-008411
<151> 2008-01-17

<160> 19

<170> PatentIn version 3.4

<210> 1
<211> 638
<212> PRT
<213> Homo sapiens

<400> 1

Met Ser Leu Gly Gln Ser Ala Cys Leu Phe Leu Ser Ile Ala Arg Ser
1               5                   10                  15

Arg Ser Val Met Thr Gly Glu Gln Met Ala Ala Phe His Pro Ser Ser
            20                  25                  30

Thr Pro Asn Pro Leu Glu Arg Pro Ile Lys Met Gly Trp Leu Lys Lys
        35                  40                  45

Gln Arg Ser Ile Val Lys Asn Trp Gln Gln Arg Tyr Phe Val Leu Arg
    50                  55                  60

Ala Gln Gln Leu Tyr Tyr Tyr Lys Asp Glu Glu Asp Thr Lys Pro Gln
65                  70                  75                  80

Gly Cys Met Tyr Leu Pro Gly Cys Thr Ile Lys Glu Ile Ala Thr Asn
                85                  90                  95

Pro Glu Glu Ala Gly Lys Phe Val Phe Glu Ile Ile Pro Ala Ser Trp
            100                 105                 110

Asp Gln Asn Arg Met Gly Gln Asp Ser Tyr Val Leu Met Ala Ser Ser
        115                 120                 125

Gln Ala Glu Met Glu Glu Trp Val Lys Phe Leu Arg Arg Val Ala Gly
    130                 135                 140

Thr Pro Cys Gly Val Phe Gly Gln Arg Leu Asp Glu Thr Val Ala Tyr
145                 150                 155                 160

Glu Gln Lys Phe Gly Pro His Leu Val Pro Ile Leu Val Glu Lys Cys
                165                 170                 175

Ala Glu Phe Ile Leu Glu His Gly Arg Asn Glu Glu Gly Ile Phe Arg
            180                 185                 190

Leu Pro Gly Gln Asp Asn Leu Val Lys Gln Leu Arg Asp Ala Phe Asp
        195                 200                 205

Ala Gly Glu Arg Pro Ser Phe Asp Arg Asp Thr Asp Val His Thr Val
    210                 215                 220

Ala Ser Leu Leu Lys Leu Tyr Leu Arg Asp Leu Pro Glu Pro Val Val
225                 230                 235                 240

Pro Trp Ser Gln Tyr Glu Gly Phe Leu Leu Cys Gly Gln Leu Thr Asn
                245                 250                 255

Ala Asp Glu Ala Lys Ala Gln Gln Glu Leu Met Lys Gln Leu Ser Ile
            260                 265                 270

Leu Pro Arg Asp Asn Tyr Ser Leu Leu Ser Tyr Ile Cys Arg Phe Leu
        275                 280                 285

His Glu Ile Gln Leu Asn Cys Ala Val Asn Lys Met Ser Val Asp Asn
    290                 295                 300

Leu Ala Thr Val Ile Gly Val Asn Leu Ile Arg Ser Lys Val Glu Asp
305                 310                 315                 320

Pro Ala Val Ile Met Arg Gly Thr Pro Gln Ile Gln Arg Val Met Thr
                325                 330                 335

Met Met Ile Arg Asp His Glu Val Leu Phe Pro Lys Ser Lys Asp Ile
            340                 345                 350

Pro Leu Ser Pro Pro Ala Gln Lys Asn Asp Pro Lys Lys Ala Pro Val
        355                 360                 365

Ala Arg Ser Ser Val Gly Trp Asp Ala Thr Glu Asp Leu Arg Ile Ser
    370                 375                 380

Arg Thr Asp Ser Phe Ser Ser Met Thr Ser Asp Ser Asp Thr Thr Ser
385                 390                 395                 400

Pro Thr Gly Gln Gln Pro Ser Asp Ala Phe Pro Glu Asp Ser Ser Lys
                405                 410                 415

Val Pro Arg Glu Lys Pro Gly Asp Trp Lys Met Gln Ser Arg Lys Arg
            420                 425                 430

Thr Gln Thr Leu Pro Asn Arg Lys Cys Phe Leu Thr Ser Ala Phe Gln
        435                 440                 445

Gly Ala Asn Ser Ser Lys Met Glu Ile Phe Lys Asn Glu Phe Trp Ser
    450                 455                 460

Pro Ser Ser Glu Ala Lys Ala Gly Glu Gly His Arg Arg Thr Met Ser
465                 470                 475                 480

Gln Asp Leu Arg Gln Leu Ser Asp Ser Gln Arg Thr Ser Thr Tyr Asp
                485                 490                 495

Asn Val Pro Ser Leu Pro Gly Ser Pro Gly Glu Glu Ala Ser Ala Leu
            500                 505                 510

Ser Ser Gln Ala Cys Asp Ser Lys Gly Asp Thr Leu Ala Ser Pro Asn
        515                 520                 525

Ser Glu Thr Gly Pro Gly Lys Lys Asn Ser Gly Glu Glu Glu Ile Asp
    530                 535                 540

Ser Leu Gln Arg Met Val Gln Glu Leu Arg Lys Glu Ile Glu Thr Gln
545                 550                 555                 560

Lys Gln Met Tyr Glu Glu Gln Ile Lys Asn Leu Glu Lys Glu Asn Tyr
                565                 570                 575

Asp Val Trp Ala Lys Val Val Arg Leu Asn Glu Glu Leu Glu Lys Glu
            580                 585                 590

Lys Lys Lys Ser Ala Ala Leu Glu Ile Ser Leu Arg Asn Met Glu Arg
        595                 600                 605

Ser Arg Glu Asp Val Glu Lys Arg Asn Lys Ala Leu Glu Glu Glu Val
    610                 615                 620

Lys Glu Phe Val Lys Ser Met Lys Glu Pro Lys Thr Glu Ala
625                 630                 635

<210>  2
<211>  572
<212>  PRT
<213>  Homo sapiens

<400>  2

Met Ala Ala Pro Arg Pro Ser Pro Ala Ile Ser Val Ser Val Ser Ala
1               5                   10                  15

Pro Ala Phe Tyr Ala Pro Gln Lys Lys Phe Gly Pro Val Val Ala Pro
            20                  25                  30

Lys Pro Lys Val Asn Pro Phe Arg Pro Gly Asp Ser Glu Pro Pro Pro
        35                  40                  45

Ala Pro Gly Ala Gln Arg Ala Gln Met Gly Arg Val Gly Glu Ile Pro
    50                  55                  60

Pro Pro Pro Pro Glu Asp Phe Pro Leu Pro Pro Pro Pro Leu Ala Gly
65                  70                  75                  80

Asp Gly Asp Asp Ala Glu Gly Ala Leu Gly Gly Ala Phe Pro Pro Pro
                85                  90                  95

Pro Pro Pro Ile Glu Glu Ser Phe Pro Pro Ala Pro Leu Glu Glu Glu
            100                 105                 110

Ile Phe Pro Ser Pro Pro Pro Pro Pro Glu Glu Glu Gly Gly Pro Glu
        115                 120                 125

Ala Pro Ile Pro Pro Pro Pro Gln Pro Arg Glu Lys Val Ser Ser Ile
    130                 135                 140

Asp Leu Glu Ile Asp Ser Leu Ser Ser Leu Leu Asp Asp Met Thr Lys
145                 150                 155                 160

Asn Asp Pro Phe Lys Ala Arg Val Ser Ser Gly Tyr Val Pro Pro Pro
                165                 170                 175

Val Ala Thr Pro Phe Ser Ser Lys Ser Ser Thr Lys Pro Ala Ala Gly
            180                 185                 190

Gly Thr Ala Pro Leu Pro Pro Trp Lys Ser Pro Ser Ser Ser Gln Pro
        195                 200                 205

Leu Pro Gln Val Pro Ala Pro Ala Gln Ser Gln Thr Gln Phe His Val
    210                 215                 220

Gln Pro Gln Pro Gln Pro Lys Pro Gln Val Gln Leu His Val Gln Ser
225                 230                 235                 240

Gln Thr Gln Pro Val Ser Leu Ala Asn Thr Gln Pro Arg Gly Pro Pro
                245                 250                 255

Ala Ser Ser Pro Ala Pro Ala Pro Lys Phe Ser Pro Val Thr Pro Lys
            260                 265                 270

Phe Thr Pro Val Ala Ser Lys Phe Ser Pro Gly Ala Pro Gly Gly Ser
        275                 280                 285

Gly Ser Gln Pro Asn Gln Lys Leu Gly His Pro Glu Ala Leu Ser Ala
    290                 295                 300

Gly Thr Gly Ser Pro Gln Pro Pro Ser Phe Thr Tyr Ala Gln Gln Arg
305                 310                 315                 320

Glu Lys Pro Arg Val Gln Glu Lys Gln His Pro Val Pro Pro Pro Ala
                325                 330                 335

Gln Asn Gln Asn Gln Val Arg Ser Pro Gly Ala Pro Gly Pro Leu Thr
            340                 345                 350

Leu Lys Glu Val Glu Glu Leu Glu Gln Leu Thr Gln Gln Leu Met Gln
        355                 360                 365

Asp Met Glu His Pro Gln Arg Gln Asn Val Ala Val Asn Glu Leu Cys
    370                 375                 380

Gly Arg Cys His Gln Pro Leu Ala Arg Ala Gln Pro Ala Val Arg Ala
385                 390                 395                 400

Leu Gly Gln Leu Phe His Ile Ala Cys Phe Thr Cys His Gln Cys Ala
                405                 410                 415

Gln Gln Leu Gln Gly Gln Gln Phe Tyr Ser Leu Glu Gly Ala Pro Tyr
            420                 425                 430

Cys Glu Gly Cys Tyr Thr Asp Thr Leu Glu Lys Cys Asn Thr Cys Gly
        435                 440                 445

Glu Pro Ile Thr Asp Arg Met Leu Arg Ala Thr Gly Lys Ala Tyr His
    450                 455                 460

Pro His Cys Phe Thr Cys Val Val Cys Ala Arg Pro Leu Glu Gly Thr
465                 470                 475                 480

Ser Phe Ile Val Asp Gln Ala Asn Arg Pro His Cys Val Pro Asp Tyr
                485                 490                 495

His Lys Gln Tyr Ala Pro Arg Cys Ser Val Cys Ser Glu Pro Ile Met
            500                 505                 510

Pro Glu Pro Gly Arg Asp Glu Thr Val Arg Val Val Ala Leu Asp Lys
        515                 520                 525

Asn Phe His Met Lys Cys Tyr Lys Cys Glu Asp Cys Gly Lys Pro Leu
    530                 535                 540

Ser Ile Glu Ala Asp Asp Asn Gly Cys Phe Pro Leu Asp Gly His Val
545                 550                 555                 560

Leu Cys Arg Lys Cys His Thr Ala Arg Ala Gln Thr
                565                 570

<210>  3
<211>  380
<212>  PRT

Seite 5

<213>  Homo sapiens

<400>  3

Met Ser Glu Thr Val Ile Cys Ser Ser Arg Ala Thr Val Met Leu Tyr
1               5                   10                  15

Asp Asp Gly Asn Lys Arg Trp Leu Pro Ala Gly Thr Gly Pro Gln Ala
            20                  25                  30

Phe Ser Arg Val Gln Ile Tyr His Asn Pro Thr Ala Asn Ser Phe Arg
        35                  40                  45

Val Val Gly Arg Lys Met Gln Pro Asp Gln Gln Val Val Ile Asn Cys
    50                  55                  60

Ala Ile Val Arg Gly Val Lys Tyr Asn Gln Ala Thr Pro Asn Phe His
65                  70                  75                  80

Gln Trp Arg Asp Ala Arg Gln Val Trp Gly Leu Asn Phe Gly Ser Lys
                85                  90                  95

Glu Asp Ala Ala Gln Phe Ala Ala Gly Met Ala Ser Ala Leu Glu Ala
            100                 105                 110

Leu Glu Gly Gly Gly Pro Pro Pro Pro Pro Ala Leu Pro Thr Trp Ser
        115                 120                 125

Val Pro Asn Gly Pro Ser Pro Glu Glu Val Glu Gln Gln Lys Arg Gln
    130                 135                 140

Gln Pro Gly Pro Ser Glu His Ile Glu Arg Arg Val Ser Asn Ala Gly
145                 150                 155                 160

Gly Pro Pro Ala Pro Pro Ala Gly Gly Pro Pro Pro Pro Pro Gly Pro
                165                 170                 175

Pro Pro Pro Pro Gly Pro Pro Pro Pro Pro Gly Leu Pro Pro Ser Gly
            180                 185                 190

Val Pro Ala Ala Ala His Gly Ala Gly Gly Gly Pro Pro Pro Ala Pro
        195                 200                 205

Pro Leu Pro Ala Ala Gln Gly Pro Gly Gly Gly Gly Ala Gly Ala Pro
    210                 215                 220

Gly Leu Ala Ala Ala Ile Ala Gly Ala Lys Leu Arg Lys Val Ser Lys
225                 230                 235                 240

Gln Glu Glu Ala Ser Gly Gly Pro Thr Ala Pro Lys Ala Glu Ser Gly
                245                 250                 255

Arg Ser Gly Gly Gly Gly Leu Met Glu Glu Met Asn Ala Met Leu Ala
            260                 265                 270

Arg Arg Arg Lys Ala Thr Gln Val Gly Glu Lys Thr Pro Lys Asp Glu
        275                 280                 285

Ser Ala Asn Gln Glu Glu Pro Glu Ala Arg Val Pro Ala Gln Ser Glu
    290                 295                 300

Ser Val Arg Arg Pro Trp Glu Lys Asn Ser Thr Thr Leu Pro Arg Met
305                 310                 315                 320

Lys Ser Ser Ser Ser Val Thr Thr Ser Glu Thr Gln Pro Cys Thr Pro
                325                 330                 335

Ser Ser Ser Asp Tyr Ser Asp Leu Gln Arg Val Lys Gln Glu Leu Leu
            340                 345                 350

Glu Glu Val Lys Lys Glu Leu Gln Lys Val Lys Glu Glu Ile Ile Glu
        355                 360                 365

Ala Phe Val Gln Glu Leu Arg Lys Arg Gly Ser Pro
    370                 375                 380

<210> 4
<211> 201
<212> PRT
<213> Homo sapiens

<400> 4

Met Lys Trp Val Trp Ala Leu Leu Leu Leu Ala Ala Leu Gly Ser Gly
1               5                   10                  15

Arg Ala Glu Arg Asp Cys Arg Val Ser Ser Phe Arg Val Lys Glu Asn
            20                  25                  30

Phe Asp Lys Ala Arg Phe Ser Gly Thr Trp Tyr Ala Met Ala Lys Lys
        35                  40                  45

Asp Pro Glu Gly Leu Phe Leu Gln Asp Asn Ile Val Ala Glu Phe Ser
    50                  55                  60

Val Asp Glu Thr Gly Gln Met Ser Ala Thr Ala Lys Gly Arg Val Arg
65                  70                  75                  80

Leu Leu Asn Asn Trp Asp Val Cys Ala Asp Met Val Gly Thr Phe Thr
                85                  90                  95

Asp Thr Glu Asp Pro Ala Lys Phe Lys Met Lys Tyr Trp Gly Val Ala
            100                 105                 110

Ser Phe Leu Gln Lys Gly Asn Asp Asp His Trp Ile Val Asp Thr Asp

        115                 120                 125

Tyr Asp Thr Tyr Ala Val Gln Tyr Ser Cys Arg Leu Leu Asn Leu Asp
    130                 135                 140

Gly Thr Cys Ala Asp Ser Tyr Ser Phe Val Phe Ser Arg Asp Pro Asn
145                 150                 155                 160

Gly Leu Pro Pro Glu Ala Gln Lys Ile Val Arg Gln Arg Gln Glu Glu
                165                 170                 175

Leu Cys Leu Ala Arg Gln Tyr Arg Leu Ile Val His Asn Gly Tyr Cys
            180                 185                 190

Asp Gly Arg Ser Glu Arg Asn Leu Leu
        195                 200

<210> 5
<211> 491
<212> PRT
<213> Homo sapiens

<400> 5

Met Ala Leu Leu Trp Gly Leu Leu Val Leu Ser Trp Ser Cys Leu Gln
1               5                   10                  15

Gly Pro Cys Ser Val Phe Ser Pro Val Ser Ala Met Glu Pro Leu Gly
            20                  25                  30

Arg Gln Leu Thr Ser Gly Pro Asn Gln Glu Gln Val Ser Pro Leu Thr
        35                  40                  45

Leu Leu Lys Leu Gly Asn Gln Glu Pro Gly Gly Gln Thr Ala Leu Lys
    50                  55                  60

Ser Pro Pro Gly Val Cys Ser Arg Asp Pro Thr Pro Glu Gln Thr His
65                  70                  75                  80

Arg Leu Ala Arg Ala Met Met Ala Phe Thr Ala Asp Leu Phe Ser Leu
                85                  90                  95

Val Ala Gln Thr Ser Thr Cys Pro Asn Leu Ile Leu Ser Pro Leu Ser
            100                 105                 110

Val Ala Leu Ala Leu Ser His Leu Ala Leu Gly Ala Gln Asn His Thr
        115                 120                 125

Leu Gln Arg Leu Gln Gln Val Leu His Ala Gly Ser Gly Pro Cys Leu
    130                 135                 140

Pro His Leu Leu Ser Arg Leu Cys Gln Asp Leu Gly Pro Gly Ala Phe
145                 150                 155                 160

Arg Leu Ala Ala Arg Met Tyr Leu Gln Lys Gly Phe Pro Ile Lys Glu
                165                 170                 175

Asp Phe Leu Glu Gln Ser Glu Gln Leu Phe Gly Ala Lys Pro Val Ser
            180                 185                 190

Leu Thr Gly Lys Gln Glu Asp Asp Leu Ala Asn Ile Asn Gln Trp Val
        195                 200                 205

Lys Glu Ala Thr Glu Gly Lys Ile Gln Glu Phe Leu Ser Gly Leu Pro
    210                 215                 220

Glu Asp Thr Val Leu Leu Leu Leu Asn Ala Ile His Phe Gln Gly Phe
225                 230                 235                 240

Trp Arg Asn Lys Phe Asp Pro Ser Leu Thr Gln Arg Asp Ser Phe His
                245                 250                 255

Leu Asp Glu Gln Phe Thr Val Pro Val Glu Met Met Gln Ala Arg Thr
            260                 265                 270

Tyr Pro Leu Arg Trp Phe Leu Leu Glu Gln Pro Glu Ile Gln Val Ala
        275                 280                 285

His Phe Pro Phe Lys Asn Asn Met Ser Phe Val Val Leu Val Pro Thr
    290                 295                 300

His Phe Glu Trp Asn Val Ser Gln Val Leu Ala Asn Leu Ser Trp Asp
305                 310                 315                 320

Thr Leu His Pro Pro Leu Val Trp Glu Arg Pro Thr Lys Val Arg Leu
                325                 330                 335

Pro Lys Leu Tyr Leu Lys His Gln Met Asp Leu Val Ala Thr Leu Ser
            340                 345                 350

Gln Leu Gly Leu Gln Glu Leu Phe Gln Ala Pro Asp Leu Arg Gly Ile
        355                 360                 365

Ser Glu Gln Ser Leu Val Val Ser Gly Val Gln His Gln Ser Thr Leu
    370                 375                 380

Glu Leu Ser Glu Val Gly Val Glu Ala Ala Ala Ala Thr Ser Ile Ala
385                 390                 395                 400

Met Ser Arg Met Ser Leu Ser Ser Phe Ser Val Asn Arg Pro Phe Leu
                405                 410                 415

Phe Phe Ile Phe Glu Asp Thr Thr Gly Leu Pro Leu Phe Val Gly Ser
            420                 425                 430

Val Arg Asn Pro Asn Pro Ser Ala Pro Arg Glu Leu Lys Glu Gln Gln
        435                 440                 445

Asp Ser Pro Gly Asn Lys Asp Phe Leu Gln Ser Leu Lys Gly Phe Pro
    450                 455                 460

Arg Gly Asp Lys Leu Phe Gly Pro Asp Leu Lys Leu Val Pro Pro Met
465                 470                 475                 480

Glu Glu Asp Tyr Pro Gln Phe Gly Ser Pro Lys
                485                 490

<210> 6
<211> 201
<212> PRT
<213> Homo sapiens

<400> 6

Met Thr Glu Lys Ala Pro Glu Pro His Val Glu Glu Asp Asp Asp Asp
1               5                   10                  15

Glu Leu Asp Ser Lys Leu Asn Tyr Lys Pro Pro Pro Gln Lys Ser Leu
            20                  25                  30

Lys Glu Leu Gln Glu Met Asp Lys Asp Asp Glu Ser Leu Ile Lys Tyr
        35                  40                  45

Lys Lys Thr Leu Leu Gly Asp Gly Pro Val Val Thr Asp Pro Lys Ala
    50                  55                  60

Pro Asn Val Val Val Thr Arg Leu Thr Leu Val Cys Glu Ser Ala Pro
65                  70                  75                  80

Gly Pro Ile Thr Met Asp Leu Thr Gly Asp Leu Glu Ala Leu Lys Lys
                85                  90                  95

Glu Thr Ile Val Leu Lys Glu Gly Ser Glu Tyr Arg Val Lys Ile His
            100                 105                 110

Phe Lys Val Asn Arg Asp Ile Val Ser Gly Leu Lys Tyr Val Gln His
        115                 120                 125

Thr Tyr Arg Thr Gly Val Lys Val Asp Lys Ala Thr Phe Met Val Gly
    130                 135                 140

Ser Tyr Gly Pro Arg Pro Glu Glu Tyr Glu Phe Leu Thr Pro Val Glu
145                 150                 155                 160

Glu Ala Pro Lys Gly Met Leu Ala Arg Gly Thr Tyr His Asn Lys Ser
                165                 170                 175

Phe Phe Thr Asp Asp Asp Lys Gln Asp His Leu Ser Trp Glu Trp Asn
            180                 185                 190

Leu Ser Ile Lys Lys Glu Trp Thr Glu
        195                 200

<210> 7
<211> 461
<212> PRT
<213> Homo sapiens

<400> 7

Met Pro Pro Ser Gly Pro Arg Gly Thr Leu Leu Leu Leu Pro Leu Leu
1               5                   10                  15

Leu Leu Leu Leu Leu Arg Ala Val Leu Ala Val Pro Leu Glu Arg Gly
            20                  25                  30

Ala Pro Asn Lys Glu Glu Thr Pro Ala Thr Glu Ser Pro Asp Thr Gly
        35                  40                  45

Leu Tyr Tyr His Arg Tyr Leu Gln Glu Val Ile Asp Val Leu Glu Thr
    50                  55                  60

Asp Gly His Phe Arg Glu Lys Leu Gln Ala Ala Asn Ala Glu Asp Ile
65                  70                  75                  80

Lys Ser Gly Lys Leu Ser Arg Glu Leu Asp Phe Val Ser His His Val
                85                  90                  95

Arg Thr Lys Leu Asp Glu Leu Lys Arg Gln Glu Val Ser Arg Leu Arg
            100                 105                 110

Met Leu Leu Lys Ala Lys Met Asp Ala Glu Gln Asp Pro Asn Val Gln
        115                 120                 125

Val Asp His Leu Asn Leu Leu Lys Gln Phe Glu His Leu Asp Pro Gln
    130                 135                 140

Asn Gln His Thr Phe Glu Ala Arg Asp Leu Glu Leu Leu Ile Gln Thr
145                 150                 155                 160

Ala Thr Arg Asp Leu Ala Gln Tyr Asp Ala Ala His His Glu Glu Phe
                165                 170                 175

Lys Arg Tyr Glu Met Leu Lys Glu His Glu Arg Arg Arg Tyr Leu Glu
            180                 185                 190

Ser Leu Gly Glu Glu Gln Arg Lys Glu Ala Glu Arg Lys Leu Glu Glu
        195                 200                 205

Gln Gln Arg Arg His Arg Glu His Pro Lys Val Asn Val Pro Gly Ser
    210                 215                 220

Gln Ala Gln Leu Lys Glu Val Trp Glu Glu Leu Asp Gly Leu Asp Pro
225                 230                 235                 240

Asn Arg Phe Asn Pro Lys Thr Phe Phe Ile Leu His Asp Ile Asn Ser
                245                 250                 255

Asp Gly Val Leu Asp Glu Gln Glu Leu Glu Ala Leu Phe Thr Lys Glu
            260                 265                 270

Leu Glu Lys Val Tyr Asp Pro Lys Asn Glu Glu Asp Asp Met Arg Glu
        275                 280                 285

Met Glu Glu Glu Arg Leu Arg Met Arg Glu His Val Met Lys Asn Val
    290                 295                 300

Asp Thr Asn Gln Asp Arg Leu Val Thr Leu Glu Glu Phe Leu Ala Ser
305                 310                 315                 320

Thr Gln Arg Lys Glu Phe Gly Asp Thr Gly Glu Gly Trp Glu Thr Val
                325                 330                 335

Glu Met His Pro Ala Tyr Thr Glu Glu Glu Leu Arg Arg Phe Glu Glu
            340                 345                 350

Glu Leu Ala Ala Arg Glu Ala Glu Leu Asn Ala Lys Ala Gln Arg Leu
        355                 360                 365

Ser Gln Glu Thr Glu Ala Leu Gly Arg Ser Gln Gly Arg Leu Glu Ala
    370                 375                 380

Gln Lys Arg Glu Leu Gln Gln Ala Val Leu His Met Glu Gln Arg Lys
385                 390                 395                 400

Gln Gln Gln Gln Gln Gln Gln Gly His Lys Ala Pro Ala Ala His Pro
                405                 410                 415

Glu Gly Gln Leu Lys Phe His Pro Asp Thr Asp Asp Val Pro Val Pro
            420                 425                 430

Ala Pro Ala Gly Asp Gln Lys Glu Val Asp Thr Ser Glu Lys Lys Leu
        435                 440                 445

Leu Glu Arg Leu Pro Glu Val Glu Val Pro Gln His Leu
    450                 455                 460

<210>  8
<211>  15
<212>  PRT
<213>  Homo sapiens
                                        Seite 12

<400> 8

Met Ser Leu Gly Gln Ser Ala Cys Leu Phe Leu Ser Ile Ala Arg
1               5                   10                  15


<210> 9
<211> 19
<212> PRT
<213> Homo sapiens

<400> 9

Val Asn Pro Phe Arg Pro Gly Asp Ser Glu Pro Pro Pro Ala Pro Gly
1               5                   10                  15


Ala Gln Arg



<210> 10
<211> 17
<212> PRT
<213> Homo sapiens

<400> 10

Ser Ser Thr Lys Pro Ala Ala Gly Gly Thr Ala Pro Leu Pro Pro Trp
1               5                   10                  15


Lys



<210> 11
<211> 12
<212> PRT
<213> Homo sapiens

<400> 11

Gly Pro Pro Ala Ser Ser Pro Ala Pro Ala Pro Lys
1               5                   10


<210> 12
<211> 11
<212> PRT
<213> Homo sapiens

<400> 12

Ser Pro Gly Ala Pro Gly Pro Leu Thr Leu Lys
1               5                   10


<210> 13
<211> 16
<212> PRT
<213> Homo sapiens

<400> 13

Leu Val Pro Pro Met Glu Glu Asp Tyr Pro Gln Phe Gly Ser Pro Lys
1               5                   10                  15

<210>  14
<211>  17
<212>  PRT
<213>  Homo sapiens

<400>  14

Ala Pro Glu Pro His Val Glu Glu Asp Asp Asp Asp Glu Leu Asp Ser
1               5                   10                  15

Lys

<210>  15
<211>  9
<212>  PRT
<213>  Homo sapiens

<400>  15

Leu Asn Tyr Lys Pro Pro Pro Gln Lys
1               5

<210>  16
<211>  10
<212>  PRT
<213>  Homo sapiens

<400>  16

Leu Pro Glu Val Glu Val Pro Gln His Leu
1               5                   10

<210>  17
<211>  97
<212>  PRT
<213>  Homo sapiens

<400>  17

Met Ser Leu Gly Gln Ser Ala Cys Leu Phe Leu Ser Ile Ala Arg Ser
1               5                   10                  15

Arg Ser Val Met Thr Gly Glu Gln Met Ala Ala Phe His Pro Ser Ser
            20                  25                  30

Thr Pro Asn Pro Leu Glu Arg Pro Ile Lys Met Gly Trp Leu Lys Lys
        35                  40                  45

Gln Arg Ser Ile Val Lys Asn Trp Gln Gln Arg Tyr Phe Val Leu Arg
    50                  55                  60

Ala Gln Gln Leu Tyr Tyr Tyr Lys Asp Glu Glu Asp Thr Lys Pro Gln
65                  70                  75                  80

Gly Cys Met Tyr Leu Pro Gly Cys Thr Ile Lys Glu Ile Ala Thr Asn
                85                  90                  95

Pro

<210> 18
<211> 30
<212> DNA
<213> Homo sapiens

<400> 18
cgcggatccc tttgctcact gccccatgtc 30

<210> 19
<211> 30
<212> DNA
<213> Homo sapiens

<400> 19
ctcgaattct tatgggtttg tggcgatctc 30

SEQUENCE LISTING

<110>  Toray Industries, Inc.

<120>  Compositions and methods for diagnosis or detection of renal
       cancer

<130>  PH-3815-PCT

<150>  JP 2008-008411
<151>  2008-01-17

<160>  19

<170>  PatentIn version 3.4

<210>  1
<211>  638
<212>  PRT
<213>  Homo sapiens

<400>  1

Met Ser Leu Gly Gln Ser Ala Cys Leu Phe Leu Ser Ile Ala Arg Ser
1               5                   10                  15

Arg Ser Val Met Thr Gly Glu Gln Met Ala Ala Phe His Pro Ser Ser
            20                  25                  30

Thr Pro Asn Pro Leu Glu Arg Pro Ile Lys Met Gly Trp Leu Lys Lys
        35                  40                  45

Gln Arg Ser Ile Val Lys Asn Trp Gln Gln Arg Tyr Phe Val Leu Arg
    50                  55                  60

Ala Gln Gln Leu Tyr Tyr Tyr Lys Asp Glu Glu Asp Thr Lys Pro Gln
65                  70                  75                  80

Gly Cys Met Tyr Leu Pro Gly Cys Thr Ile Lys Glu Ile Ala Thr Asn
                85                  90                  95

Pro Glu Glu Ala Gly Lys Phe Val Phe Glu Ile Ile Pro Ala Ser Trp
            100                 105                 110

Asp Gln Asn Arg Met Gly Gln Asp Ser Tyr Val Leu Met Ala Ser Ser
        115                 120                 125

Gln Ala Glu Met Glu Glu Trp Val Lys Phe Leu Arg Arg Val Ala Gly
    130                 135                 140

Thr Pro Cys Gly Val Phe Gly Gln Arg Leu Asp Glu Thr Val Ala Tyr
145                 150                 155                 160

Glu Gln Lys Phe Gly Pro His Leu Val Pro Ile Leu Val Glu Lys Cys
                165                 170                 175

Ala Glu Phe Ile Leu Glu His Gly Arg Asn Glu Glu Gly Ile Phe Arg
            180                 185                 190

Leu Pro Gly Gln Asp Asn Leu Val Lys Gln Leu Arg Asp Ala Phe Asp
        195                 200                 205

Ala Gly Glu Arg Pro Ser Phe Asp Arg Asp Thr Asp Val His Thr Val
    210                 215                 220

Ala Ser Leu Leu Lys Leu Tyr Leu Arg Asp Leu Pro Glu Pro Val Val
225                 230                 235                 240

Pro Trp Ser Gln Tyr Glu Gly Phe Leu Leu Cys Gly Gln Leu Thr Asn
                245                 250                 255

Ala Asp Glu Ala Lys Ala Gln Gln Glu Leu Met Lys Gln Leu Ser Ile
            260                 265                 270

Leu Pro Arg Asp Asn Tyr Ser Leu Leu Ser Tyr Ile Cys Arg Phe Leu
        275                 280                 285

His Glu Ile Gln Leu Asn Cys Ala Val Asn Lys Met Ser Val Asp Asn
    290                 295                 300

Leu Ala Thr Val Ile Gly Val Asn Leu Ile Arg Ser Lys Val Glu Asp
305                 310                 315                 320

Pro Ala Val Ile Met Arg Gly Thr Pro Gln Ile Gln Arg Val Met Thr
                325                 330                 335

Met Met Ile Arg Asp His Glu Val Leu Phe Pro Lys Ser Lys Asp Ile
            340                 345                 350

Pro Leu Ser Pro Pro Ala Gln Lys Asn Asp Pro Lys Lys Ala Pro Val
        355                 360                 365

Ala Arg Ser Ser Val Gly Trp Asp Ala Thr Glu Asp Leu Arg Ile Ser
    370                 375                 380

Arg Thr Asp Ser Phe Ser Ser Met Thr Ser Asp Ser Asp Thr Thr Ser
385                 390                 395                 400

Pro Thr Gly Gln Gln Pro Ser Asp Ala Phe Pro Glu Asp Ser Ser Lys
                405                 410                 415

Val Pro Arg Glu Lys Pro Gly Asp Trp Lys Met Gln Ser Arg Lys Arg
            420                 425                 430

Thr Gln Thr Leu Pro Asn Arg Lys Cys Phe Leu Thr Ser Ala Phe Gln
        435                 440                 445

Gly Ala Asn Ser Ser Lys Met Glu Ile Phe Lys Asn Glu Phe Trp Ser
    450                 455                 460

Pro Ser Ser Glu Ala Lys Ala Gly Glu Gly His Arg Arg Thr Met Ser
465                 470                 475                 480

Gln Asp Leu Arg Gln Leu Ser Asp Ser Gln Arg Thr Ser Thr Tyr Asp
                485                 490                 495

Asn Val Pro Ser Leu Pro Gly Ser Pro Gly Glu Glu Ala Ser Ala Leu
            500                 505                 510

Ser Ser Gln Ala Cys Asp Ser Lys Gly Asp Thr Leu Ala Ser Pro Asn
        515                 520                 525

Ser Glu Thr Gly Pro Gly Lys Lys Asn Ser Gly Glu Glu Glu Ile Asp
    530                 535                 540

Ser Leu Gln Arg Met Val Gln Glu Leu Arg Lys Glu Ile Glu Thr Gln
545                 550                 555                 560

Lys Gln Met Tyr Glu Glu Gln Ile Lys Asn Leu Glu Lys Glu Asn Tyr
                565                 570                 575

Asp Val Trp Ala Lys Val Val Arg Leu Asn Glu Glu Leu Glu Lys Glu
            580                 585                 590

Lys Lys Lys Ser Ala Ala Leu Glu Ile Ser Leu Arg Asn Met Glu Arg
        595                 600                 605

Ser Arg Glu Asp Val Glu Lys Arg Asn Lys Ala Leu Glu Glu Glu Val
    610                 615                 620

Lys Glu Phe Val Lys Ser Met Lys Glu Pro Lys Thr Glu Ala
625                 630                 635

<210>  2
<211>  572
<212>  PRT
<213>  Homo sapiens

<400>  2

Met Ala Ala Pro Arg Pro Ser Pro Ala Ile Ser Val Ser Val Ser Ala
1               5                   10                  15

Pro Ala Phe Tyr Ala Pro Gln Lys Lys Phe Gly Pro Val Val Ala Pro
            20                  25                  30

Lys Pro Lys Val Asn Pro Phe Arg Pro Gly Asp Ser Glu Pro Pro Pro
        35                  40                  45

Ala Pro Gly Ala Gln Arg Ala Gln Met Gly Arg Val Gly Glu Ile Pro
    50                  55                  60

Pro Pro Pro Pro Glu Asp Phe Pro Leu Pro Pro Pro Pro Leu Ala Gly
65                  70                  75                  80

Asp Gly Asp Asp Ala Glu Gly Ala Leu Gly Gly Ala Phe Pro Pro Pro
                85                  90                  95

Pro Pro Pro Ile Glu Glu Ser Phe Pro Pro Ala Pro Leu Glu Glu Glu
            100                 105                 110

Ile Phe Pro Ser Pro Pro Pro Pro Pro Glu Glu Glu Gly Gly Pro Glu
        115                 120                 125

Ala Pro Ile Pro Pro Pro Pro Gln Pro Arg Glu Lys Val Ser Ser Ile
    130                 135                 140

Asp Leu Glu Ile Asp Ser Leu Ser Ser Leu Leu Asp Asp Met Thr Lys
145                 150                 155                 160

Asn Asp Pro Phe Lys Ala Arg Val Ser Ser Gly Tyr Val Pro Pro Pro
                165                 170                 175

Val Ala Thr Pro Phe Ser Ser Lys Ser Ser Thr Lys Pro Ala Ala Gly
            180                 185                 190

Gly Thr Ala Pro Leu Pro Pro Trp Lys Ser Pro Ser Ser Ser Gln Pro
        195                 200                 205

Leu Pro Gln Val Pro Ala Pro Ala Gln Ser Gln Thr Gln Phe His Val
    210                 215                 220

Gln Pro Gln Pro Gln Pro Lys Pro Gln Val Gln Leu His Val Gln Ser
225                 230                 235                 240

Gln Thr Gln Pro Val Ser Leu Ala Asn Thr Gln Pro Arg Gly Pro Pro
                245                 250                 255

Ala Ser Ser Pro Ala Pro Ala Pro Lys Phe Ser Pro Val Thr Pro Lys
            260                 265                 270

Phe Thr Pro Val Ala Ser Lys Phe Ser Pro Gly Ala Pro Gly Gly Ser
        275                 280                 285

Gly Ser Gln Pro Asn Gln Lys Leu Gly His Pro Glu Ala Leu Ser Ala
    290                 295                 300

Gly Thr Gly Ser Pro Gln Pro Pro Ser Phe Thr Tyr Ala Gln Gln Arg
305                 310                 315                 320

Glu Lys Pro Arg Val Gln Glu Lys Gln His Pro Val Pro Pro Pro Ala
                325                 330                 335

Gln Asn Gln Asn Gln Val Arg Ser Pro Gly Ala Pro Gly Pro Leu Thr
            340                 345                 350

Leu Lys Glu Val Glu Glu Leu Glu Gln Leu Thr Gln Gln Leu Met Gln
        355                 360                 365

Asp Met Glu His Pro Gln Arg Gln Asn Val Ala Val Asn Glu Leu Cys
    370                 375                 380

Gly Arg Cys His Gln Pro Leu Ala Arg Ala Gln Pro Ala Val Arg Ala
385                 390                 395                 400

Leu Gly Gln Leu Phe His Ile Ala Cys Phe Thr Cys His Gln Cys Ala
                405                 410                 415

Gln Gln Leu Gln Gly Gln Gln Phe Tyr Ser Leu Glu Gly Ala Pro Tyr
            420                 425                 430

Cys Glu Gly Cys Tyr Thr Asp Thr Leu Glu Lys Cys Asn Thr Cys Gly
        435                 440                 445

Glu Pro Ile Thr Asp Arg Met Leu Arg Ala Thr Gly Lys Ala Tyr His
    450                 455                 460

Pro His Cys Phe Thr Cys Val Val Cys Ala Arg Pro Leu Glu Gly Thr
465                 470                 475                 480

Ser Phe Ile Val Asp Gln Ala Asn Arg Pro His Cys Val Pro Asp Tyr
                485                 490                 495

His Lys Gln Tyr Ala Pro Arg Cys Ser Val Cys Ser Glu Pro Ile Met
            500                 505                 510

Pro Glu Pro Gly Arg Asp Glu Thr Val Arg Val Val Ala Leu Asp Lys
        515                 520                 525

Asn Phe His Met Lys Cys Tyr Lys Cys Glu Asp Cys Gly Lys Pro Leu
    530                 535                 540

Ser Ile Glu Ala Asp Asp Asn Gly Cys Phe Pro Leu Asp Gly His Val
545                 550                 555                 560

Leu Cys Arg Lys Cys His Thr Ala Arg Ala Gln Thr
                565                 570


<210>  3
<211>  380
<212>  PRT
<213>  Homo sapiens

<400>  3
   Met Ser Glu Thr Val Ile Cys Ser Ser Arg Ala Thr Val Met Leu Tyr
   1               5                   10                  15
   Asp Asp Gly Asn Lys Arg Trp Leu Pro Ala Gly Thr Gly Pro Gln Ala
               20                  25                  30
   Phe Ser Arg Val Gln Ile Tyr His Asn Pro Thr Ala Asn Ser Phe Arg
           35                  40                  45
   Val Val Gly Arg Lys Met Gln Pro Asp Gln Gln Val Val Ile Asn Cys
       50                  55                  60
   Ala Ile Val Arg Gly Val Lys Tyr Asn Gln Ala Thr Pro Asn Phe His
   65                  70                  75                  80
   Gln Trp Arg Asp Ala Arg Gln Val Trp Gly Leu Asn Phe Gly Ser Lys
                   85                  90                  95
   Glu Asp Ala Ala Gln Phe Ala Ala Gly Met Ala Ser Ala Leu Glu Ala
   100 105 110
   Leu Glu Gly Gly Gly Pro Pro Pro Pro Pro Ala Leu Pro Thr Trp Ser
   115 120 125
   Val Pro Asn Gly Pro Ser Pro Glu Glu Val Glu Gln Gln Lys Arg Gln
   130 135 140
   Gln Pro Gly Pro Ser Glu His Ile Glu Arg Arg Val Ser Asn Ala Gly
   145 150 155 160
   Gly Pro Pro Ala Pro Pro Ala Gly Gly Pro Pro Pro Pro Pro Gly Pro
   165 170 175
   Pro Pro Pro Pro Gly Pro Pro Pro Pro Pro Gly Leu Pro Pro Ser Gly
   180 185 190
   Val Pro Ala Ala Ala His Gly Ala Gly Gly Gly Pro Pro Pro Ala Pro
   195 200 205
   Pro Leu Pro Ala Ala Gln Gly Pro Gly Gly Gly Gly Ala Gly Ala Pro
   210 215 220
   Gly Leu Ala Ala Ala Ile Ala Gly Ala Lys Leu Arg Lys Val Ser Lys
   225 230 235 240
   Gln Glu Glu Ala Ser Gly Gly Pro Thr Ala Pro Lys Ala Glu Ser Gly
   245 250 255
   Arg Ser Gly Gly Gly Gly Leu Met Glu Glu Met Asn Ala Met Leu Ala
   260 265 270
   Arg Arg Arg Lys Ala Thr Gln Val Gly Glu Lys Thr Pro Lys Asp Glu
   275 280 285
   Ser Ala Asn Gln Glu Glu Pro Glu Ala Arg Val Pro Ala Gln Ser Glu
   290 295 300
   Ser Val Arg Arg Pro Trp Glu Lys Asn Ser Thr Thr Leu Pro Arg Met
   305 310 315 320
   Lys Ser Ser Ser Ser Val Thr Thr Ser Glu Thr Gln Pro Cys Thr Pro
   325 330 335
   Ser Ser Ser Asp Tyr Ser Asp Leu Gln Arg Val Lys Gln Glu Leu Leu
   340 345 350
   Glu Glu Val Lys Lys Glu Leu Gln Lys Val Lys Glu Glu Ile Ile Glu
           355                 360                 365
   Ala Phe Val Gln Glu Leu Arg Lys Arg Gly Ser Pro
       370                 375                 380

<210>  4
<211>  201
<212>  PRT
<213>  Homo sapiens

<400>  4

Met Lys Trp Val Trp Ala Leu Leu Leu Leu Ala Ala Leu Gly Ser Gly
1               5                   10                  15

Arg Ala Glu Arg Asp Cys Arg Val Ser Ser Phe Arg Val Lys Glu Asn
            20                  25                  30

Phe Asp Lys Ala Arg Phe Ser Gly Thr Trp Tyr Ala Met Ala Lys Lys
        35                  40                  45

Asp Pro Glu Gly Leu Phe Leu Gln Asp Asn Ile Val Ala Glu Phe Ser
    50                  55                  60

Val Asp Glu Thr Gly Gln Met Ser Ala Thr Ala Lys Gly Arg Val Arg
65                  70                  75                  80

Leu Leu Asn Asn Trp Asp Val Cys Ala Asp Met Val Gly Thr Phe Thr
                85                  90                  95

Asp Thr Glu Asp Pro Ala Lys Phe Lys Met Lys Tyr Trp Gly Val Ala
            100                 105                 110

Ser Phe Leu Gln Lys Gly Asn Asp Asp His Trp Ile Val Asp Thr Asp
        115                 120                 125

Tyr Asp Thr Tyr Ala Val Gln Tyr Ser Cys Arg Leu Leu Asn Leu Asp
    130                 135                 140

Gly Thr Cys Ala Asp Ser Tyr Ser Phe Val Phe Ser Arg Asp Pro Asn
145                 150                 155                 160

Gly Leu Pro Pro Glu Ala Gln Lys Ile Val Arg Gln Arg Gln Glu Glu
                165                 170                 175

Leu Cys Leu Ala Arg Gln Tyr Arg Leu Ile Val His Asn Gly Tyr Cys
            180                 185                 190

Asp Gly Arg Ser Glu Arg Asn Leu Leu
        195                 200

<210>  5
<211>  491
<212>  PRT
<213>  Homo sapiens

<400>  5

Met Ala Leu Leu Trp Gly Leu Leu Val Leu Ser Trp Ser Cys Leu Gln
1               5                   10                  15

Gly Pro Cys Ser Val Phe Ser Pro Val Ser Ala Met Glu Pro Leu Gly
            20                  25                  30

Arg Gln Leu Thr Ser Gly Pro Asn Gln Glu Gln Val Ser Pro Leu Thr
        35                  40                  45

Leu Leu Lys Leu Gly Asn Gln Glu Pro Gly Gly Gln Thr Ala Leu Lys
    50                  55                  60

Ser Pro Pro Gly Val Cys Ser Arg Asp Pro Thr Pro Glu Gln Thr His
65                  70                  75                  80

Arg Leu Ala Arg Ala Met Met Ala Phe Thr Ala Asp Leu Phe Ser Leu
                85                  90                  95

Val Ala Gln Thr Ser Thr Cys Pro Asn Leu Ile Leu Ser Pro Leu Ser
            100                 105                 110

Val Ala Leu Ala Leu Ser His Leu Ala Leu Gly Ala Gln Asn His Thr
        115                 120                 125

Leu Gln Arg Leu Gln Gln Val Leu His Ala Gly Ser Gly Pro Cys Leu
    130                 135                 140

Pro His Leu Leu Ser Arg Leu Cys Gln Asp Leu Gly Pro Gly Ala Phe
145                 150                 155                 160

Arg Leu Ala Ala Arg Met Tyr Leu Gln Lys Gly Phe Pro Ile Lys Glu
                165                 170                 175

Asp Phe Leu Glu Gln Ser Glu Gln Leu Phe Gly Ala Lys Pro Val Ser
            180                 185                 190

Leu Thr Gly Lys Gln Glu Asp Asp Leu Ala Asn Ile Asn Gln Trp Val
        195                 200                 205

Lys Glu Ala Thr Glu Gly Lys Ile Gln Glu Phe Leu Ser Gly Leu Pro
    210                 215                 220

Glu Asp Thr Val Leu Leu Leu Leu Asn Ala Ile His Phe Gln Gly Phe
225                 230                 235                 240

Trp Arg Asn Lys Phe Asp Pro Ser Leu Thr Gln Arg Asp Ser Phe His
                245                 250                 255

Leu Asp Glu Gln Phe Thr Val Pro Val Glu Met Met Gln Ala Arg Thr
            260                 265                 270

Tyr Pro Leu Arg Trp Phe Leu Leu Glu Gln Pro Glu Ile Gln Val Ala
        275                 280                 285

His Phe Pro Phe Lys Asn Asn Met Ser Phe Val Val Leu Val Pro Thr
    290                 295                 300

His Phe Glu Trp Asn Val Ser Gln Val Leu Ala Asn Leu Ser Trp Asp
305                 310                 315                 320

Thr Leu His Pro Pro Leu Val Trp Glu Arg Pro Thr Lys Val Arg Leu
                325                 330                 335

Pro Lys Leu Tyr Leu Lys His Gln Met Asp Leu Val Ala Thr Leu Ser
            340                 345                 350

Gln Leu Gly Leu Gln Glu Leu Phe Gln Ala Pro Asp Leu Arg Gly Ile
        355                 360                 365

Ser Glu Gln Ser Leu Val Val Ser Gly Val Gln His Gln Ser Thr Leu
    370                 375                 380

Glu Leu Ser Glu Val Gly Val Glu Ala Ala Ala Ala Thr Ser Ile Ala
385                 390                 395                 400

Met Ser Arg Met Ser Leu Ser Ser Phe Ser Val Asn Arg Pro Phe Leu
                405                 410                 415

Phe Phe Ile Phe Glu Asp Thr Thr Gly Leu Pro Leu Phe Val Gly Ser
            420                 425                 430

Val Arg Asn Pro Asn Pro Ser Ala Pro Arg Glu Leu Lys Glu Gln Gln
        435                 440                 445

Asp Ser Pro Gly Asn Lys Asp Phe Leu Gln Ser Leu Lys Gly Phe Pro
    450                 455                 460

Arg Gly Asp Lys Leu Phe Gly Pro Asp Leu Lys Leu Val Pro Pro Met
465                 470                 475                 480

Glu Glu Asp Tyr Pro Gln Phe Gly Ser Pro Lys
                485                 490

<210>  6
<211>  201
<212>  PRT
<213>  Homo sapiens

<400>  6

Met Thr Glu Lys Ala Pro Glu Pro His Val Glu Glu Asp Asp Asp Asp
1               5                   10                  15

Glu Leu Asp Ser Lys Leu Asn Tyr Lys Pro Pro Pro Gln Lys Ser Leu
            20                  25                  30

Lys Glu Leu Gln Glu Met Asp Lys Asp Asp Glu Ser Leu Ile Lys Tyr
        35                  40                  45

Lys Lys Thr Leu Leu Gly Asp Gly Pro Val Val Thr Asp Pro Lys Ala
    50                  55                  60

Pro Asn Val Val Val Thr Arg Leu Thr Leu Val Cys Glu Ser Ala Pro
65                  70                  75                  80

Gly Pro Ile Thr Met Asp Leu Thr Gly Asp Leu Glu Ala Leu Lys Lys
                85                  90                  95

Glu Thr Ile Val Leu Lys Glu Gly Ser Glu Tyr Arg Val Lys Ile His
            100                 105                 110

Phe Lys Val Asn Arg Asp Ile Val Ser Gly Leu Lys Tyr Val Gln His
        115                 120                 125

Thr Tyr Arg Thr Gly Val Lys Val Asp Lys Ala Thr Phe Met Val Gly
    130                 135                 140

Ser Tyr Gly Pro Arg Pro Glu Glu Tyr Glu Phe Leu Thr Pro Val Glu

145                 150                 155                 160

Glu Ala Pro Lys Gly Met Leu Ala Arg Gly Thr Tyr His Asn Lys Ser
                165                 170                 175

Phe Phe Thr Asp Asp Asp Lys Gln Asp His Leu Ser Trp Glu Trp Asn
            180                 185                 190

Leu Ser Ile Lys Lys Glu Trp Thr Glu
        195                 200

<210>  7
<211>  461
<212>  PRT
<213>  Homo sapiens

<400>  7

Met Pro Pro Ser Gly Pro Arg Gly Thr Leu Leu Leu Leu Pro Leu Leu
1               5                   10                  15

Leu Leu Leu Leu Leu Arg Ala Val Leu Ala Val Pro Leu Glu Arg Gly
            20                  25                  30

Ala Pro Asn Lys Glu Glu Thr Pro Ala Thr Glu Ser Pro Asp Thr Gly
        35                  40                  45

Leu Tyr Tyr His Arg Tyr Leu Gln Glu Val Ile Asp Val Leu Glu Thr
    50                  55                  60

Asp Gly His Phe Arg Glu Lys Leu Gln Ala Ala Asn Ala Glu Asp Ile
65                  70                  75                  80

Lys Ser Gly Lys Leu Ser Arg Glu Leu Asp Phe Val Ser His His Val
                85                  90                  95

Arg Thr Lys Leu Asp Glu Leu Lys Arg Gln Glu Val Ser Arg Leu Arg
            100                 105                 110

Met Leu Leu Lys Ala Lys Met Asp Ala Glu Gln Asp Pro Asn Val Gln
        115                 120                 125

Val Asp His Leu Asn Leu Leu Lys Gln Phe Glu His Leu Asp Pro Gln
    130                 135                 140

Asn Gln His Thr Phe Glu Ala Arg Asp Leu Glu Leu Leu Ile Gln Thr
145                 150                 155                 160

Ala Thr Arg Asp Leu Ala Gln Tyr Asp Ala Ala His His Glu Glu Phe
                165                 170                 175

Lys Arg Tyr Glu Met Leu Lys Glu His Glu Arg Arg Arg Tyr Leu Glu
            180                 185                 190

Ser Leu Gly Glu Glu Gln Arg Lys Glu Ala Glu Arg Lys Leu Glu Glu
        195                 200                 205

Gln Gln Arg Arg His Arg Glu His Pro Lys Val Asn Val Pro Gly Ser
    210                 215                 220

Gln Ala Gln Leu Lys Glu Val Trp Glu Glu Leu Asp Gly Leu Asp Pro
225                 230                 235                 240

Asn Arg Phe Asn Pro Lys Thr Phe Phe Ile Leu His Asp Ile Asn Ser
                245                 250                 255

Asp Gly Val Leu Asp Glu Gln Glu Leu Glu Ala Leu Phe Thr Lys Glu
            260                 265                 270

Leu Glu Lys Val Tyr Asp Pro Lys Asn Glu Glu Asp Asp Met Arg Glu
        275                 280                 285

Met Glu Glu Glu Arg Leu Arg Met Arg Glu His Val Met Lys Asn Val
    290                 295                 300

Asp Thr Asn Gln Asp Arg Leu Val Thr Leu Glu Glu Phe Leu Ala Ser
305                 310                 315                 320

Thr Gln Arg Lys Glu Phe Gly Asp Thr Gly Glu Gly Trp Glu Thr Val
                325                 330                 335

Glu Met His Pro Ala Tyr Thr Glu Glu Glu Leu Arg Arg Phe Glu Glu
            340                 345                 350

Glu Leu Ala Ala Arg Glu Ala Glu Leu Asn Ala Lys Ala Gln Arg Leu
        355                 360                 365

Ser Gln Glu Thr Glu Ala Leu Gly Arg Ser Gln Gly Arg Leu Glu Ala
    370                 375                 380

Gln Lys Arg Glu Leu Gln Gln Ala Val Leu His Met Glu Gln Arg Lys
385                 390                 395                 400

Gln Gln Gln Gln Gln Gln Gln Gly His Lys Ala Pro Ala Ala His Pro
                405                 410                 415

Glu Gly Gln Leu Lys Phe His Pro Asp Thr Asp Asp Val Pro Val Pro
            420                 425                 430

Ala Pro Ala Gly Asp Gln Lys Glu Val Asp Thr Ser Glu Lys Lys Leu
        435                 440                 445

Leu Glu Arg Leu Pro Glu Val Glu Val Pro Gln His Leu
    450                 455                 460

<210>  8
<211>  15
<212>  PRT
<213>  Homo sapiens

<400>  8

Met Ser Leu Gly Gln Ser Ala Cys Leu Phe Leu Ser Ile Ala Arg
1               5                   10                  15

<210>  9
<211>  19
<212>  PRT
<213>  Homo sapiens

<400>  9

Val Asn Pro Phe Arg Pro Gly Asp Ser Glu Pro Pro Pro Ala Pro Gly
1               5                   10                  15

Ala Gln Arg

<210>  10
<211>  17
<212>  PRT
<213>  Homo sapiens

<400>  10

Ser Ser Thr Lys Pro Ala Ala Gly Gly Thr Ala Pro Leu Pro Pro Trp
1               5                   10                  15

Lys

<210>  11
<211>  12
<212>  PRT
<213>  Homo sapiens

<400>  11

Gly Pro Pro Ala Ser Ser Pro Ala Pro Ala Pro Lys
1               5                   10


<210>  12
<211>  11
<212>  PRT
<213>  Homo sapiens

<400>  12

Ser Pro Gly Ala Pro Gly Pro Leu Thr Leu Lys
1               5                   10


<210>  13
<211>  16
<212>  PRT
<213>  Homo sapiens

<400>  13

Leu Val Pro Pro Met Glu Glu Asp Tyr Pro Gln Phe Gly Ser Pro Lys
1               5                   10                  15


<210>  14
<211>  17
<212>  PRT
<213>  Homo sapiens

<400>  14

Ala Pro Glu Pro His Val Glu Glu Asp Asp Asp Asp Glu Leu Asp Ser
1               5                   10                  15


Lys



<210>  15
<211>  9
<212>  PRT
<213>  Homo sapiens

<400>  15

Leu Asn Tyr Lys Pro Pro Pro Gln Lys
1               5


<210>  16
<211>  10
<212>  PRT
<213>  Homo sapiens

<400>  16

Leu Pro Glu Val Glu Val Pro Gln His Leu
1               5                   10

<210>  17
<211>  97
<212>  PRT
<213>  Homo sapiens

<400>  17

Met Ser Leu Gly Gln Ser Ala Cys Leu Phe Leu Ser Ile Ala Arg Ser
1               5                   10                  15

Arg Ser Val Met Thr Gly Glu Gln Met Ala Ala Phe His Pro Ser Ser
            20                  25                  30

Thr Pro Asn Pro Leu Glu Arg Pro Ile Lys Met Gly Trp Leu Lys Lys
        35                  40                  45

Gln Arg Ser Ile Val Lys Asn Trp Gln Gln Arg Tyr Phe Val Leu Arg
    50                  55                  60

Ala Gln Gln Leu Tyr Tyr Tyr Lys Asp Glu Glu Asp Thr Lys Pro Gln
65                  70                  75                  80

Gly Cys Met Tyr Leu Pro Gly Cys Thr Ile Lys Glu Ile Ala Thr Asn
                85                  90                  95

Pro

<210>  18
<211>  30
<212>  DNA
<213>  Homo sapiens

<400>  18
cgcggatccc tttgctcact gccccatgtc                                        30

<210>  19
<211>  30
<212>  DNA
<213>  Homo sapiens

<400>  19
ctcgaattct tatgggtttg tggcgatctc                                        30